(19) Europäisches Patentamt
European Patent Office
Office européen des brevets

(11) Numéro de publication: **0 406 502 B1**

# (12) FASCICULE DE BREVET EUROPEEN

(45) Date de publication de fascicule du brevet: **10.05.95**

(51) Int. Cl.[6]: **C07D 211/90**, A61K 31/445

(21) Numéro de dépôt: **89402702.8**

(22) Date de dépôt: **02.10.89**

Le dossier contient des informations techniques présentées postérieurement au dépôt de la demande et ne figurant pas dans le présent fascicule.

## (54) Procédé de préparation de la (-) [[(amino-2 éthoxy)-2 éthoxy]méthyl]-2 (dichloro-2,3 phényl)-4 éthoxycarbonyl-3 méthoxycarbonyl-5 méthyl-6 dihydro-1,4 pyridine, et intermédiaire pour sa préparation.

(30) Priorité: **04.07.89 FR 8908920**

(43) Date de publication de la demande:
**09.01.91 Bulletin 91/02**

(45) Mention de la délivrance du brevet:
**10.05.95 Bulletin 95/19**

(84) Etats contractants désignés:
**AT BE CH DE ES FR GB GR IT LI LU NL SE**

(56) Documents cités:
**EP-A- 0 089 167**
**EP-A- 0 259 206**

**JOURNAL OF MEDICINAL CHEMISTRY, vol. 29, 1986, pages 1696-1702, AmericanChemical Society; J.E. ARROWSMITH et al.: "Long-acting dihydropyridine calciumantagonists. 1. 2-alkoxymethyl derivatives incorporating basic substituents"**

(73) Titulaire: **ADIR ET COMPAGNIE**
**1 rue Carle Hébert**
**F-92415 Courbevoie Cédex (FR)**

(72) Inventeur: **Peglion, Jean-Louis**
**5 Allée des Bégonias**
**F-78110 Le Vesinet (FR)**
Inventeur: **Gargouil, Yves-Michel**
**38 Rue Michel Ange**
**F-75016 Paris (FR)**
Inventeur: **Vilaine, Jean-Paul**
**3 Avenue des Fusillés**
**F-92290 Chatenay Malabry (FR)**

EP 0 406 502 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de préparation d'un dérivé de la dihydro-1,4 pyridine, la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine et de ses sels.

Certains dérivés de la dihydro-1,4 pyridine possèdant d'intéressantes propriétés pharmacologiques ont été décrits dans le brevet EP 259206. Ce brevet décrit et revendique, parmi un grand nombre de composés, l'{[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine. Le procédé d'obtention de ce composé sous sa forme racémique est exemplifié dans ce brevet.

Compte-tenu des résultats thérapeutiques très intéressants obtenus sur ce produit, des recherches plus approfondies ont été entreprises pour séparer les isomères optiques de ce composé et ont abouti au procédé de préparation de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine, procédé qui fait l'objet de la présente demande.

En effet, les différents procédés de séparations d'isomères ou de synthèses stéréosélectives décrits dans la littérature ne permettent pas l'obtention des isomères de l'{[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine. Il a donc été nécessaire d'inventer un nouveau procédé.

Ce procédé, objet de la présente invention, n'entre pas dans les procédés classiques antérieurement connus pour préparer des composés analogues. D'autre part, si l'on se réfère au procédé décrit dans J. Med. Chem. vol 29 n° 9, 1696-1702 (1986), considéré comme l'art antérieur le plus proche, on remarquera que les produits, préparés selon cet art antérieur, contiennent tous, jusqu'au stade final, une fonction azide ($N_3$) particulièrement connue comme cause d'explosion notamment dans le domaine industriel. Un tel risque n'existe pas dans le procédé de la présente invention (aucun des produits initiaux, finaux ou intermédiaires ne renfermant de fonction azide).

La (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine est un puissant inhibiteur de la pénétration intracellulaire du calcium.

Theoriquement, l'isomère actif d'un composé doué de propriétés pharmacologiques, possède au maximum le double de l'activité par rapport au racémique.

Dans le cas présent, les essais pharmacologiques ont démontré que activité au composé préparé selon l'invention est très superieure.

La présente invention a plus particulièrement pour objet le procédé de préparation de la :
(-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine composé de formule I :

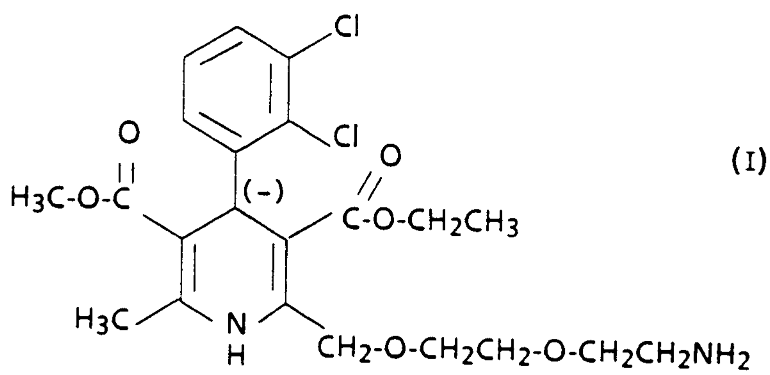

(I)

et de ses sels d'addition avec un acide minéral ou organique pharmaceutiquement acceptable.

Parmi les acides utilisés, on peut citer les acides fumarique, tartrique, camphosulfonique, chlorhydrique, citrique, méthane sulfonique, benzène sulfonique etc...

La présente invention a pour objet le procédé de préparation du composé de formule I,

caractérisé en ce que l'on condense le [(chloro-2 éthoxy)-2 éthoxy]-2 éthanol avec la phtalimide potassique, dans la diméthylformamide à chaud, pour former le [(phtalimido-2 éthoxy)-2 éthoxy]-2 éthanol, composé de formule II,

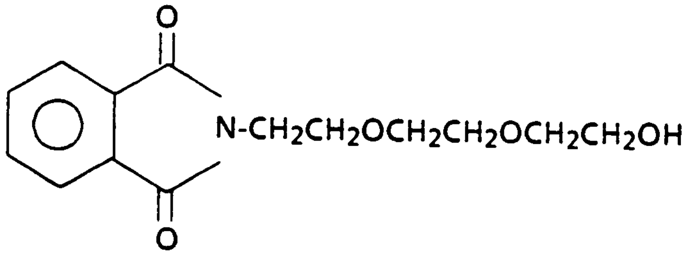

(II)

que l'on transforme à l'aide du réactif de Jones en acide [(phtalimido-2 éthoxy)-2 éthoxy]-2 acétique, composé de formule III:

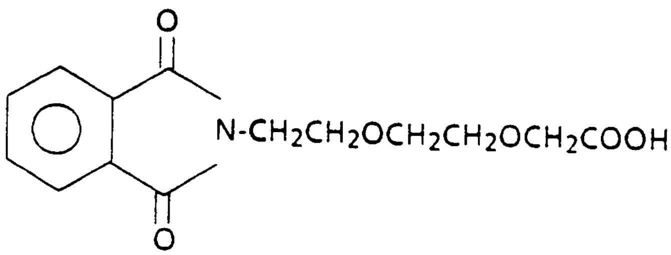

(III)

que l'on traite avec le carbonyldiimidazole et l'acide de Meldrum en présence de pyridine dans le chlorure de méthylène, pour obtenir le composé de formule IV:

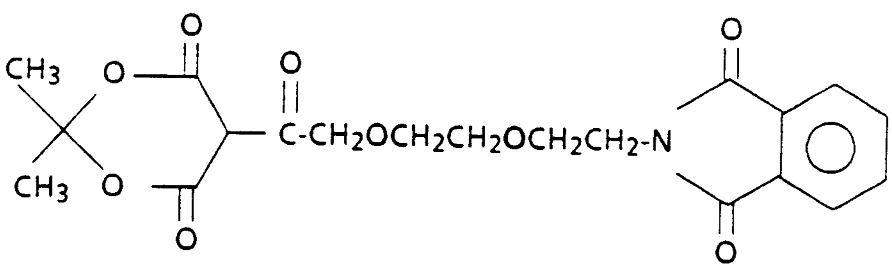

(IV)

lequel ensuite est mis à réagir avec le (R) phényl-2 méthoxy-2 éthanol, composé de formule V :

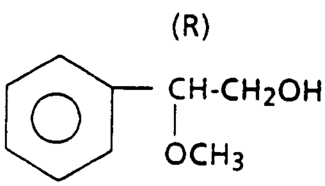

(V)

pour obtenir le $\beta$-cétoester de formule VI:

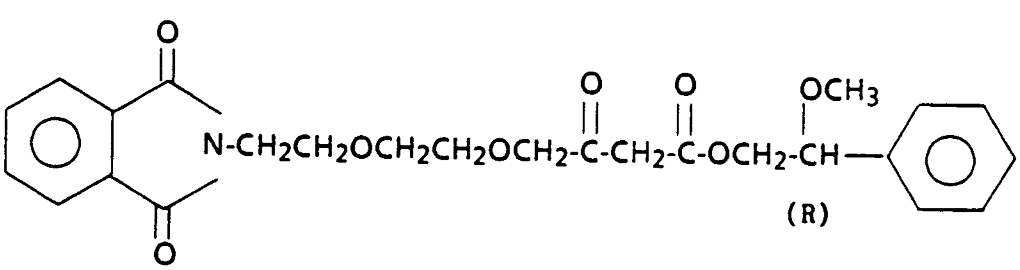

(VI)

qu'on condense en présence de formiate d'ammonium dans l'éthanol avec un benzylidène de formule VII:

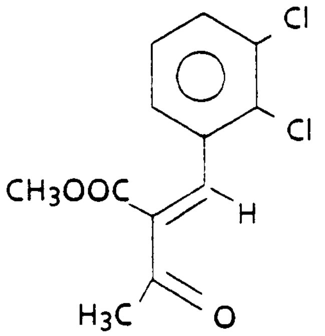

(VII)

pour obtenir la (4R4'R/4S4'R) (dichloro-2,3 phényl)-4 méthoxycarbonyl-5 (méthoxy-2 phényl-2 éthoxycarbonyl)-3 méthyl-6 {[(phtalimido-2 éthoxy)-2 éthoxy] méthyl}-2 dihydro-1,4 pyridine, composé de formule VIII:

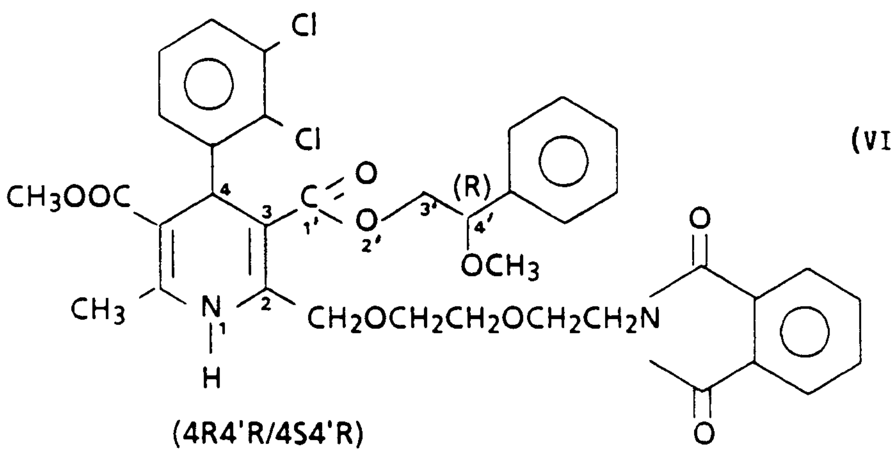

(VIII)

(4R4'R/4S4'R)

laquelle ensuite est

soit:

soumise à l'action d'une solution aqueuse de bicarbonate de sodium, en solution dans l'acétonitrile, pour obtenir la (4R4'R/4S4'R) {[[(carboxy-2 phénylcarboxamido)-2 éthoxy)]-2 éthoxy] méthyl}-2 (dichloro-2,3 phényl)-4 méthoxycarbonyl-5 (méthoxy-2 phényl-2 éthoxycarbonyl)-3 méthyl-6 dihydro-1,4 pyridine, composé de formule IX:

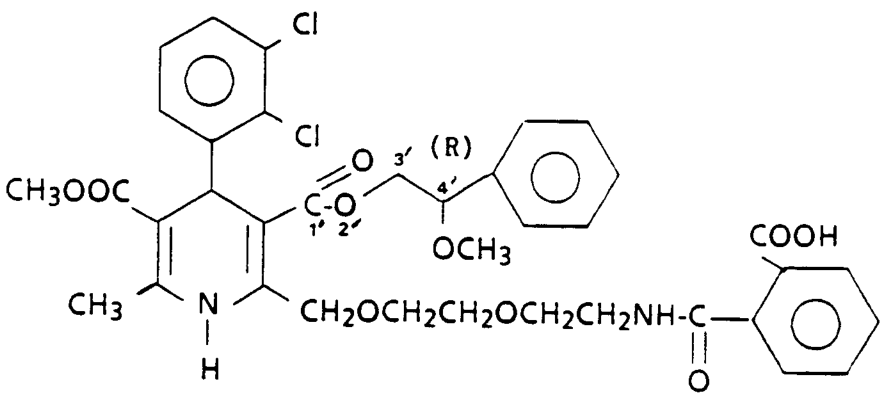

(IX)

(4R4'R/4S4'R)

que l'on traite, après séparation par HPLC, par un mélange de glyme et d'éthylate de sodium, pour obtenir un mélange contenant la (-) {[[(carboxy-2 phénylcarboxamido)-2 éthoxy]-2 éthoxy] méthyl}-2 (dichloro-2,3 phényl)-4 éthoxycarbonyl-3 méthoxycarbonyl-5 méthyl-6 dihydro-1,4 pyridine, composé de formule X,

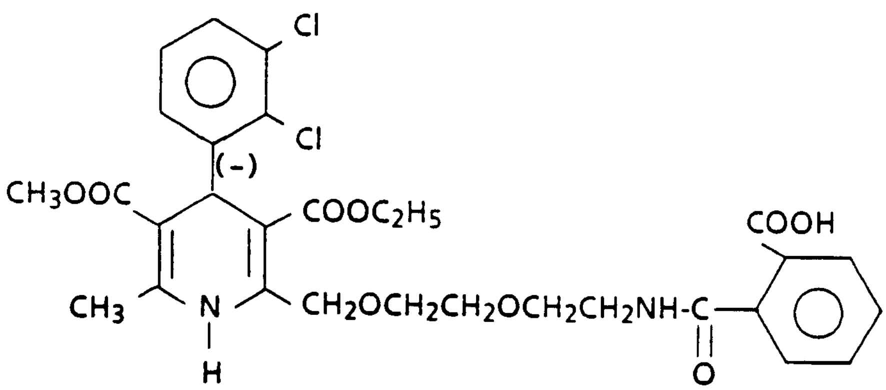

(X)

et son homologue, substitué en position 5 par un radical éthoxycarbonyle,

soit:

séparée par chromatographie sur colonne de silice en utilisant comme éluant un mélange de chlorure de méthylène et d'acétate d'éthyle (95:5 V/V) pour obtenir l'isomère le moins polaire de la (dichloro-2,3 phényl)-4 méthoxycarbonyl-5 (méthoxy-2 phényl-2 éthoxycarbonyl)-3 méthyl-6 {[(phtalimido-2 éthoxy)-2 éthoxy] méthyl}-2 dihydro-1,4 pyridine, lequel ensuite est soumis à l'action d'éthanolate de sodium en présence de glyme en solution dans l'éthanol, pour obtenir un mélange contenant le composé de formule X et son homologue, substitué en position 5 par un radical éthoxycarbonyle, lequel est ensuite soumis à l'action du carbonyldiimidazole en solution dans un alcane halogéné à température ambiante pour obtenir un mélange contenant de la (-) (dichloro-2,3 phényl)-4 éthoxycarbonyl-3 méthoxycarbonyl-5 méthyl-6 {[-(phtalimido-2 éthoxy)-2 éthoxy] méthyl}-2 dihydro-1,4 pyridine composé de formule XI:

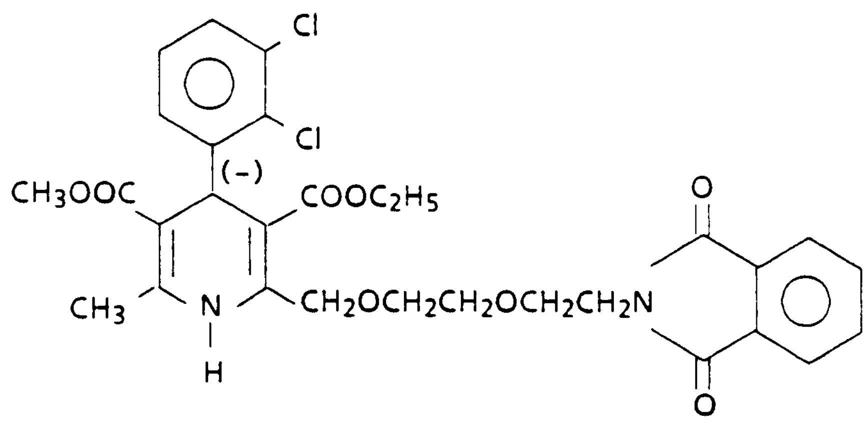

(XI)

et son homologue substitué en 5 par un radical éthoxycarbonyle lequel mélange est ensuite séparé par HPLC, pour obtenir la (-) (dichloro-2,3 phényl)-4 éthoxycarbonyl-3 méthoxycarbonyl-5 méthyl-6 {[-(phtalimido-2 éthoxy)-2 éthoxy] méthyl}-2 dihydro-1,4 pyridine pure,
laquelle est ensuite portée au reflux dans l'éthanol en présence d'hydrate d'hydrazine pour donner le composé de la formule I qui peut être ensuite salifié avec un acide organique ou minéral pharmaceutiquement compatible.

Le (R) phényl-2 méthoxy-2 éthanol composé de formule Y est obtenu par réduction de l'acide correspondant, optiquement actif. Ce dernier composé est préparé selon le procédé décrit dans J.Chem.Soc.,1962,p.1519.

L'ester méthylique de l'acide (dichloro-2,3 benzylidène)-2 oxo-3 butanoïque, composé de formule VII est obtenu par condensation du dichloro-2,3 benzaldéhyde avec l'acétoacétate de méthyle.

Le composé de formule I, est un inhibiteur de la pénétration intra-cellulaire du calcium nettement plus puissant que son racémique correspondant. En effet, de manière surprenante, les résultats des essais pharmacologiques ont démontré que le composé de formule I possède une activité qui est de 4 à 30 fois supérieure par rapport à l'activité du racémique.

La concentration en calcium intracellulaire joue un rôle de messager pour de nombreuses fonctions biologiques : contractions, sécrétions; cette concentration dépend largement de la pénétration transmembranaire en calcium, qui, très concentrée dans les milieux extracellulaires, transite à travers les canaux sélectifs pour le calcium situés dans la membrane.

C'est ainsi que des inhibiteurs de ces canaux limitant ou supprimant la pénétration du calcium peuvent avoir des effets thérapeutiques intéressants dans un nombre de pathologies comme par exemple vasorelaxation, pour le traitement de l'hypertension artérielle et l'hypertension pulmonaire, des maladies vasculaires périphériques et coronaires. (Am.J.Card.1980,<u>46</u>,p.1047-1058; Burger's Medicinal Chemistry 4ème Edition, Part III,p.5456 - John Wiley and Sons Inc. USA., 1981; Life Sciences, 1983,<u>33</u>,p.2571-2581). Des effets bénéfiques induits sont également constatés pour le traitement des insuffisances cardiaques.

La modération de la contraction myocardique est aussi utile dans les situations ischémiques cardiaques. (Medicine, 1985,<u>64</u>,p.61-73). La limitation de la pénétration du calcium dans les cellules peut également jouer un rôle important pour prévenir l'accumulation calcique caractéristique du vieillisement cellulaire et liée à certaines maladies dégénératives vasculaires -athéromatoses en particulier- (Medicinal Research Review, 1985,5,p.394-425).

La modulation calcique présente également un intérêt pour le traitement de l'épilepsie et des vertiges d'origine centrale. La limitation du calcium ionisé dans les fibres lisses des parois du tube digestif permet aussi la levée des spasmes oesophagiens et au niveau pulmonaire celui du spasme bronchique (traitement de l'asthme). Cette modération du calcium peut aussi être utile comme adjuvant dans les traitements du cancer et de l'hypercoagulation.

La présente description n'est d'ailleurs pas limitative, les travaux fondamentaux mettent en effet l'accent sur le rôle de premier plan joué par le calcium dans nombre de phénomènes physiologiques et physiopathologiques.

Les essais pharmacologiques chez le rat, ont prouvé in vivo, que l'activité du composé de l'invention est supérieure à celle de son racémique et qu'il est doué d'une durée d'action plus longue et confirment l'intérêt de son emploi en thérapeutique.

L'invention s'étend aussi à la préparation des compositions pharmaceutiques renfermant comme principe actif le composé de formule I, ou un de ses sels d'addition avec un acide minéral ou organique

avec un ou plusieurs excipients, inertes, non toxiques et appropriés.

Les compositions pharmaceutiques ainsi obtenues sont présentées avantageusement sous des formes diverses telles que par exemple comprimés, dragées, gélules, glossettes ou autres préparations galéniques appropriées.

Ces compositions pharmaceutiques peuvent en outre contenir un principe actif d'action complémentaire ou synergique.

La posologie peut varier largement en fonction de l'âge, du poids du patient, de la nature et de la sévérité de l'affection ainsi que de la voie buccale ou parentérale. D'une manière générale, la posologie unitaire s'échelonnera entre 0,02 et 50 mg et la posologie journalière administrée par voie orale, utilisable en thérapeutique humaine ou animale entre 0,02 et 100 mg.

Les exemple suivants, donnés à titre non-limitatif, illustrent l'invention.

Les points de fusion indiqués sont mesurés selon la technique micro-Köfler.

Les spectres de résonance magnétique nucléaire du proton (R.M.N.) ont été enregistrés à 200 MHz.

## EXEMPLE 1

## Acide [(phtalimido-2 éthoxy)-2 éthoxy]-2 acétique

### STADE A

[(phtalimido-2 éthoxy)-2 éthoxy]-2 éthanol

Porter à 95 ° C, pendant 17 heures, 188 g de [(chloro-2 éthoxy)-2 éthoxy]-2 éthanol, 146 g de phtalimide potassique dans 700 ml de diméthylformamide.

Diluer au chlorure de méthylène, laver par une solution saturée de chlorure de sodium, sécher, évaporer.

Distiller au Kugelrohr $Eb_{0,05\ mmHg}$ : 180-185 ° C

Rendement : 90%

Spectre de résonance magnétique nucléaire du proton (Solvant $CDCl_3$): 4H(m)7,5 à 8 ppm; 12H(m)3,4 à 4 ppm; 1H (massif échangeable $D_2O$) 2,5 à 3 ppm.

### STADE B

Dissoudre 3 g de l'alcool obtenu au stade précédent dans 150 ml d'acétone. Couler le réactif de Jones en maintenant la température entre 20 ° et 25 ° C. Laisser reposer une heure à la température ambiante. Concentrer, diluer ensuite au chlorure de méthylène, laver à l'eau. Sécher et évaporer le solvant pour obtenir le composé attendu.

Point de Fusion : 88-90 ° C

Rendement : 90%

Spectre de résonance magnétique nucléaire du proton (Solvant $CDCl_3$): 1H (massif échangeable) 8,8 à 9,5 ppm: 4H(m)7,6 à 8,1 ppm; 2H(s)4,1 ppm; 8H (m)3,6 à 4 ppm.

## EXEMPLE 2

## (R) phényl-2 méthoxy-2 éthanol

Réduire 72 g d'acide (R) phényl-2 méthoxy-2 acétique (préparé selon la méthode décrite dans J.Chem.Soc.,1962,p.1519), par 16,5 g d'hydrure de lithium et d'aluminium dans 300 ml de tétrahydrofuranne.

Hydrolyser et filtrer les sels minéraux et ensuite distiller l'huile résiduelle au Kugelrohr. $E_{b15\ mmHg}$ = 105 ° C.

Rendement : 76%

Spectre de résonance magnétique nucléaire du proton (Solvant $CDCl_3$): 5H(m) 7,35 ppm; 1H(d)4,35 ppm; 2H(m)3,65 ppm; 3H(s)3,3 ppm; 1H (d échangeable) 2,35 ppm.

| Pouvoir rotatoire en solution à 1% dans l'éthanol: | |
|---|---|
| λ(nm) | $[\alpha]^{24°C}$ |
| 589 | - 122° |
| 578 | - 127° |
| 546 | - 145° |
| 436 | - 249° |
| 365 | - 396° |

**EXEMPLE 3**

**Ester (R) phényl-2 méthoxy-2 éthylique de l'acide dioxa-5,8 oxo-3 phtalimido-10 décanoïque**

A une suspension contenant 34,7 g du composé de l'exemple 1 dans 210 ml de chlorure de méthylène on ajoute en une fois 20 g de carbonyldiimidazole.

Agiter jusqu'à fin de dégagement gazeux. Couler ensuite en un goutte à goutte rapide un mélange constitué de 17,7 g d'acide de Meldrum et 9,2 g de pyridine dans 70 ml de chlorure de méthylène. Agiter sous azote pendant une nuit.

Transvaser dans une ampoule à décanter, laver à l'acide sulfurique N jusqu'à pH acide, puis une fois à l'eau, sécher et évaporer le solvant.

Porter l'huile au bain marie avec 25 g de l'alcool obtenu dans l'exemple 2, jusqu'à fin de dégagement.

Soumettre le milieu réactionnel à une chromatographie sur colonne (chromatographie flash) contenant 1,8 kg de silice en utilisant comme éluant un mélange de cyclohexane et d'acétate d'éthyle (1:1 V/V) pour obtenir le composé attendu.

Rendement : 70%.

Spectre de résonance magnétique nucléaire du proton (solvant $CDCl_3$):

4H(m)7,6 à 8,1 ppm, 5H(s)7,35 ppm, 3H(m)4 à 4,6 ppm, 2H(s)4,1 ppm, 10H(m)3,5 à 4 ppm, 3H(s)3,3 ppm.

| Pouvoir rotatoire en solution à 1% dans l'éthanol: | |
|---|---|
| λ(nm) | $[\alpha]^{21°C}$ |
| 589 | - 35,1° |
| 578 | - 36,5° |
| 546 | - 41,4° |
| 436 | - 69,8° |
| 365 | - 107,2° |

**EXEMPLE 4**

**Ester méthylique de l'acide (dichloro-2,3 benzylidène)-2 oxo-3 butanoïque**

Porter au reflux sous agitation pendant 4 heures, un mélange contenant 8,7 g de dichloro-2,3 benzaldéhyde, 5,8 g d'acétoacétate de méthyle, 28 gouttes de pyridine et 38 gouttes d'acide hexanoïque dans 280 ml de benzène. Transvaser dans une ampoule, laver avec une solution à 10% de bicarbonate de sodium, puis avec une solution d'acide chlorhydrique N, puis à l'eau. Sécher et évaporer. Les cristaux obtenus sont lavés par l'éther isopropylique.

Rendement : 65%

Spectre de résonance magnétique nucléaire du proton (Solvant $CDCl_3$): 1H(2s)8 et 8,05 ppm; 3H(m),7,1 à 7,8 ppm; 3H(2s)3,9 et 3,75 ppm, 3H(2s) 2,45 et 2,2 ppm.

**EXEMPLE 5**

**(4R4'R/4S4'R) (dichloro-2,3 phényl)-4 méthoxycarbonyl-5 (méthoxy-2 phényl-2 éthoxycarbonyl)-3 méthyl-6 {[(phtalimido-2 éthoxy)-2 éthoxy] méthyl}-2 dihydro-1,4 pyridine**

Agiter sous azote à 40°C pendant 48 heures un mélange contenant 22,2 g du composé décrit dans l'exemple 4, 38 g du composé décrit dans l'exemple 3, et 6,3 g de formiate d'ammonium dans 200 ml d'éthanol. Le milieu résiduel est évaporé et purifié sur une colonne contenant 4 kg de silice en utilisant comme éluant un mélange constitué de chlorure de méthylène et d'acétate d'éthyle (9:1 V/V).
Spectre de résonance magnétique nucléaire du proton (Solvant ($CDCl_3$) : 2H(m)7,8 ppm; 2H(m)7,7 ppm; 7H(m)7,3 à 7,1 ppm, 1H(t)7,05 ppm; 1H(s)5,45 ppm; 2H(m)4,6 ppm, 11H(m)3,6 à 4,4 ppm; 3H(2s)3,6 ppm; 3H(2s)3,2 et 3,05 ppm; 3H(s)2,3 ppm; 1H(s) non échangeable par $D_2O$, 7,4 ppm.

| Pouvoir rotatoire en solution à 1% dans le chloroforme: | |
|---|---|
| **λ(nm)** | **$[\alpha]^{21°C}$** |
| 589 | - 13,3° |
| 578 | - 13,8° |
| 546 | - 15,1° |

**EXEMPLE 6**

**(4R4'R/4S4'R) {[[(carboxy-2 phénylcarboxamido)-2 éthoxy]-2 éthoxy] méthyl}-2 (dichloro-2,3 phényl)-4 méthoxycarbonyl-5 (méthoxy-2 phényl-2 éthoxycarbonyl)-3 méthyl-6 dihydro-1,4 pyridine**

Porter au reflux sous agitation pendant 24 heures un mélange contenant 16,5 g du composé de l'exemple 5, 100 ml de solution aqueuse à 10% de bicarbonate de sodium et 230 ml d'acétonitrile. Evaporer le solvant, reprendre à l'eau, acidifer à l'acide chlorhydrique N et extraire pour obtenir le composé attendu.
Rendement : 87%
Spectre de résonance magnétique nucléaire du proton (Solvant $CDCl_3$): 1H(m)7,9 ppm; 3H(m)7,5 ppm; 7H-(m)7,4 à 7,15 ppm; 1H(t)7,05 ppm; 1H(2s)5,45 ppm; 2H(m)4,8 ppm; 2H(m)4,4 à 4,1 ppm, 1H(m)3,9 ppm; 8H(m)3,8 à 3,5 ppm; 3H(2s)3,6 ppm; 3H(2s)3,2 et 3,05 ppm; 3H(s)2,3 ppm; 1H(t)6,55 ppm; 1H (signal masqué échangé par $D_2O$) 7,45 ppm; 1H (signal plat) 7 à 5 ppm échangé par $D_2O$.

| Pouvoir rotatoire en solution à 1% dans le chloroforme : | |
|---|---|
| **λ(nm)** | **$[\alpha]^{21°C}$** |
| 589 | - 14,2° |
| 578 | - 14,8° |
| 546 | - 16,3° |

**EXEMPLE 7**

**Mélange de (-) {[[(carboxy-2 phényl carboxamido)-2 éthoxy]-2 éthoxy] méthyl}-2 (dichloro-2,3 phényl)-4 éthoxycarbonyl-3 méthoxycarbonyl-5 méthyl-6 dihydro-1,4 pyridine et de son homologue substitué en position 5 par un radical éthoxycarbonyle**

*** Premier procédé**

Le composé de l'exemple 6 est séparé en ses deux isomères par HPLC en utilisant comme colonne une Lichroprep RP18 de 50 cm de long et comme éluant un mélange de méthanol et de phosphate

disodique 0,025M (55:45 V/V), débit 10 ml/min.

3 g du deuxième composé issu de la séparation sont portés au reflux avec 30 ml de glyme et 28,6 ml d'une solution d'éthylate de sodium 0,26M. Evaporer, reprendre à l'eau, acidifier et extraire à l'acétate d'éthyle pour obtenir les composés attendus.
Rendement global : 10%

*** Deuxième procédé**

STADE A

Le composé de l'exemple 5 est chromatographié sur une colonne préparative contenant 4 kg de silice, en utilisant comme éluant un mélange de chlorure de méthylène et d'acétate d'éthyle (95:5). Isoler le premier composé issu de la séparation qui est le moins polaire.
Rendement : 20%
Spectre de résonance magnétique nucléaire du proton (solvant $CDCl_3$) : 2H(m)7,8 ppm; 2H(m)7,7 ppm; 7H-(m)7,4 à 7,1 ppm; 1H(t)7,05 ppm; 1H(s),5,45 ppm; 2H(m)4,4 ppm; 11H(m)4 à 3,7 ppm; 3H(s)3,6 ppm; 3H-(s)3,05 ppm; 3H(s)2,3 ppm; 1H(s)7,45 ppm (difficilement échangeable par $D_2O$).

| Pouvoir rotatoire en solution à 1% dans le chloroforme: | |
|---|---|
| **λ(nm)** | **$[\alpha]^{21°C}$** |
| 589 | - 9° |
| 578 | - 9,6° |
| 546 | - 10,4° |

STADE B

Porter 3 g du composé obtenu au stade précédent à reflux avec 30 ml de glyme et 28,6 ml d'une solution éthanolique d'éthanolate de sodium 0,26M. Evaporer, reprendre à l'eau, acidifier et extraire à l'acétate d'éthyle pour obtenir les composés attendus.
Rendement : 65%

**EXEMPLE 8**

**Mélange de (-) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine et de son homologue substitué en 5 par un radical éthoxycarbonyle.**

Dissoudre 1,9 g du mélange obtenu dans l'exemple 7 dans 30 ml de chlorure de méthylène et ajouter en une fois 0,9 g de carbonyldiimidazole; Laisser sous agitation pendant une nuit.

Transvaser le milieu réactionnel dans une ampoule à décanter, laver au bicarbonate de sodium à 10% et ensuite à l'acide chlorhydrique N et à l'eau. Sécher et évaporer pour obtenir les composés attendus.
Rendement : 65%

**EXEMPLE 9**

**(-) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine**

Séparer le mélange obtenu dans l'exemple 8 par HPLC préparative en utilisant une colonne Lichroprep RP 18 de 50 cm de long et comme éluant un mélange d'éthanol, d'eau et de TFA (500:500:1). Le composé attendu est isolé en premier.
Rendement : 30%

| Pouvoir rotatoire en solution à 1% dans le DMSO : | |
|---|---|
| λ(nm) | $[\alpha]^{20°C}$ |
| 589 | - 34,6° |
| 578 | - 36,5° |
| 546 | - 43,9° |
| 436 | - 119,0° |

**EXEMPLE 10**

**(-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

Mettre à reflux 1 g du composé préparé dans l'exemple 9 avec 10 ml d'éthanol et 0,25 ml d'hydrate d'hydrazine pendant 4 heures. Evaporer le solvant, reprendre à l'éther éthylique, laver avec 5 ml de soude normale et épuiser la phase éthérée à l'acide chlorhydrique N. Les phases aqueuses sont ensuite basifiées et extraites à l'éther éthylique pour obtenir le composé attendu.
Rendement : 60%
Point de fusion: 69°-71°C.
Spectre de résonance magnétique nucléaire du proton (solvant $CDCl_3$) : 1H(m)7,3 à7,7 ppm échangeable par $D_2O$; 3H(m)7,6 à 6,9 ppm; 1H(s)5,5 ppm; 2H(s)4,8 ppm; 2H(q)4 ppm; 4H(s)3,7 ppm; 2H(m)3,4 à 3,7 ppm; 3H(s)3,6 ppm; 2H(t)2,9 ppm; 3H(s)2,3 ppm; 2H(m)échangeables par $D_2O$ 1,4 à 1,8 ppm; 3H(t)1,2 ppm.

| Pouvoir rotatoire en solution à 1% dans le chloroforme: | |
|---|---|
| λ(nm) | $[\alpha]^{20,5°C}$ |
| 589 | - 36,5° |
| 578 | - 38,7° |
| 546 | - 46,8° |
| 436 | - 133° |

**EXEMPLE 11**

**Fumarate de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

Le fumarate de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine est obtenu après solubilisation de 4,2 g de base dans 50 ml d'une solution éthanolique d'acide fumarique 0,172M. Recristallisation dans l'acétonitrile.
Rendement : 92%
Point de fusion : 115°C
Spectre de résonance magnétique nucléaire du proton (Solvant $CDCl_3$ et DMSO-$d_6$): 2H(2dd)7,3 ppm; 1H-(t)7,1 ppm; 2H(s)6,7 ppm; 1H(s)5,45 ppm; 2H(m)4,7 ppm; 2H(q)4 ppm; 6H(m)5,7 ppm; 3H(s)3,6 ppm; 2H-(m)3,1 ppm; 3H(s)2,3 ppm; 3H(t)1,3 ppm; 1H(s échangé $D_2O$)7,7 ppm; 4H(s échangé $D_2O$)5,7 ppm.

| Pouvoir rotatoire en solution à 1% dans le DMSO: | |
|---|---|
| **λ(nm)** | **$[\alpha]^{20,5°C}$** |
| 589 | - 33,1° |
| 578 | - 35,2° |
| 546 | - 43,0° |
| 436 | - 134,6° |

**EXEMPLE 12**

**(+) Tartrate de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

0,2 g du composé de l'exemple 10 est dissous dans 3,1 ml de solution éthanolique d'acide (+) tartrique 0,133M. Après évaporation du solvant, on obtient 0,24 g du sel attendu.
Point de fusion : 150°C

| Pouvoir rotatoire en solution à 1% dans le DMSO : | |
|---|---|
| **λ(nm)** | **$[\alpha]^{21,5°C}$** |
| 589 | - 29,9° |
| 578 | - 32,5° |
| 546 | - 40,5° |
| 436 | - 133,9° |

**EXEMPLE 13**

**(-) Tartrate de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

0,3 g du composé de l'exemple 10 est dissous dans 4,6 ml d'une solution éthanolique d'acide (-) tartrique 0,133M. On isole le sel attendu après filtration.
Point de fusion : 161-166°C (sublimation)

| Pouvoir rotatoire en solution à 1% dans le DMSO : | |
|---|---|
| **λ(nm)** | **$[\alpha]^{21,5°C}$** |
| 589 | - 32,9° |
| 578 | - 35° |
| 546 | - 42,8° |
| 436 | - 142,4° |

**EXEMPLE 14**

**Tartrate racémique de la (-) {[(amino-2 éthoxy)-2 éthoxy] méthyl}-2 (dichloro-2,3 phényl)-4 éthoxy-carbonyl-3 méthoxycarbonyl-5 méthyl-6 dihydro-1,4 pyridine**

0,45 g du composé de l'exemple 10 sont dissous dans 6,9 ml d'une solution éthanolique d'acide tartrique racémique 0,133M.

On isole le sel attendu après filtration.

Point de fusion : 160-170 °C

| Pouvoir rotatoire en solution à 1% dans le DMSO : | |
|---|---|
| **λ(nm)** | $[\alpha]^{21,5°C}$ |
| 589 | - 31,2 ° |
| 578 | - 33,5 ° |
| 546 | - 41,1 ° |
| 436 | - 135,9 ° |

**ETUDE PHARMACOLOGIOUE**

**EXEMPLE 15**

**Affinité pour le site de fixation des dihydropyridines**

L'étude du déplacement par la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine et par son racémique du PN 200-110 tritié, fixé de façon spécifique au site de dihydropyridines associé au canal calcique lent, a permis de démontrer que le composé de l'invention présente une affinité pour ces sites 30 fois supérieure par rapport à son racémique.

Cette étude a été réalisée sur des préparations de membranes microsomales préparées à partir de muscle squelettique des membres postérieurs de rat (Wistar).

Les rats sont tués par décapitation. Les muscles squelettiques sont prélevés et lavés dans le tampon I (MOPS/KOH pH 7,4 20mM, saccharose 0,3 M, EDTA 1 mM, iodoacétamide 0,1 mM, pepstatine A 1 µM, leupeptine 1 mg/l et PMSF 0,1 µM). Ensuite les muscles sont fragmentés mécaniquement et repris dans 4 volumes de tampon I par gramme de tissus et homogénéisés.

L'homogénat est ensuite centrifugé, pendant 10 minutes à 3.200 g les culots sont éliminés et les surnageants sont ensuite centrifugés pendant 20 minutes à 15.000 g, les surnageants sont agités durant 15 minutes à 4 °C dans le tampon I contenant 0.6 M final de KCl, puis centrifugés 45 minutes à 70 000 g.

Les culots sont repris dans le tampon I, homogénéisés au potter. Les homogénats sont centrifugés à 100 000 g durant 45 minutes et les culots ainsi obtenus correspondent à la fraction microsomale utilisée pour les études de liaison et de déplacement.

L'activité des produits est appréciée par le déplacement du [$^3$H] PN 200-110, fixé de façon spécifique à son récepteur associé au canal calcique lent par des concentrations croissantes des produits à examiner.

La valeur obtenue (K0.5), pour chacun des produits, qui représente la concentration de produit déplaçant 50% du [$^3$H] PN 200-110, permet le calcul d'une constante de dissociation vraie ($K_I$):

$$K\,0.5 = K_I \times \frac{1 + [L^*]}{Kd}$$

où [L*] est la concentration du [$^3$H] PN 200-110 libre et Kd la constante de dissociation à l'équilibre du complexe [$^3$H] PN 200-110 déterminée par fixation directe.

Les $K_I$ des deux produits sont rapportés au Tableau I :

TABLEAU I

| COMPOSE | $K_I$ EXPRIME EN nM |
|---|---|
| COMPOSE DE L'INVENTION | 0,28 ± 0,15 |
| RACEMIQUE | 8,2 ± 1,7 |

**EXEMPLE 16**

**Etude sur l'artère mésentérique isolée de lapin contractée par le calcium**

Cet essai est effectué sur des organes isolés prélevés chez le lapin mâle de Nouvelle Zélande de 2,5 à 3 kg placé sous diète hydrique 18 heures avant le sacrifice.

Après sacrifice rapide de l'animal, l'artère mésentérique est prélevée est disséquée en anneau de 2 mm de longueur ; l'endothélium est éliminé de façon mécanique.

Après une période de stabilisation de 90 minutes, les préparations sont soumises à un milieu dépolarisant (35 mM KCl) et sans calcium (0,1 mM EGTA) pendant 15 minutes.

Une gamme contrôle est réalisée en ajoutant au bain toutes les 6 minutes des doses cumulées de calcium jusqu'à l'effet maximal. Les préparations sont alors rincées pendant une période de 15 minutes et le produit est incubé une heure le renouvellement de la gamme de calcium.

Une seule concentration de produit est testée par organe.

Les réponses obtenues en présence de produit sont exprimées en pourcentage de la valeur maximale de la gamme contrôle.

Le pD′2 est calculé selon la méthode de Van Rossum (1963).

Les valeurs de pD′2 du composé de l'invention et de son racémique figurent au Tableau II. Ces données indiquent la très nette supériorité (plus que le double) du composé de l'invention, par rapport à son racémique, à prévenir une contraction vasculaire induite par le calcium.

TABLEAU II

| COMPOSE | pD′2 |
|---|---|
| COMPOSE DE L'INVENTION | 7,97 |
| RACEMIQUE | 7,64 |

**EXEMPLE 17**

**Etude chez le rat spontanément hypertendu (SHR) éveillé**

Des rats mâles SHR de 280 à 350 g, âgés de 18 à 24 semaines sont anesthésiés à l'imalgène ® (150 mg/kg i.p.). Deux cathéters en polyéthylène sont introduits, l'un dans l'aorte abdominale pour l'enregistrement de paramètres hémodynamiques, l'autre dans la veine jugulaire pour l'administration des produits.
Les cathéters sont tunellisés au niveau de cou. Les animaux sont utilisés au moins 48 heures après intervention.

Les pressions artérielles abdominales systolique et diastolique sont enregistrées à l'aide d'un capteur Statham P23XL sur un enregistreur Gould ES 1000. Les produits sont administrés par voie intraveineuse sous un volume de 0,5 ml/kg. Les doses administrées des composés étudiés sont exprimées en mg/kg de base. La pression artérielle est enregistrée en continu jusqu'à 6 heures après le traitement.

Les variations de pression artérielle systolique après traitement sont reportées dans le Tableau III.

Le composé de l'invention se distingue de son racémique par son activité plus puissante et encore mieux soutenue dans le temps, ce qui constitue un avantage considérable en thérapeutique. En effet, pour la même dose six heures après traitement, l'activité du composé de l'invention est environ 4 fois supérieure par rapport à son racémique.

TABLEAU III

| COMPOSE | DOSE (mg/kg) | Variations de pression artérielle systolique (ΔmmHg) après traitement | | |
|---|---|---|---|---|
| | | 1H30 | 3H | 6H |
| COMPOSE DE L'INVENTION | 0,1 | -16±2 | -18±2 | -21±5 |
| RACEMIQUE | 0,1 | -11±3 | -5±3 | -5±2 |

**PREPARATION PHARMACEUTIOUE**

**EXEMPLE 18**

**Comprimés dosés à 1 mg de (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine (A.D.E.M.M.D.P.):**

| | |
|---|---|
| **A.D.E.M.M.D.P.** | 1 mg |
| **Saccharose amidon** | 64 mg |
| **Cellulose excipient** | 25 mg |
| **Acide alginique** | 10 mg |

pour un comprimé d'un poids théorique de 100,00 mg.

**Revendications**

**Revendications pour les Etats contractants suivants : AT, BE, CH, DE, FR, GB, IT, LI, LU, NL, SE**

**1.** Procédé de préparation du composé de formule I :

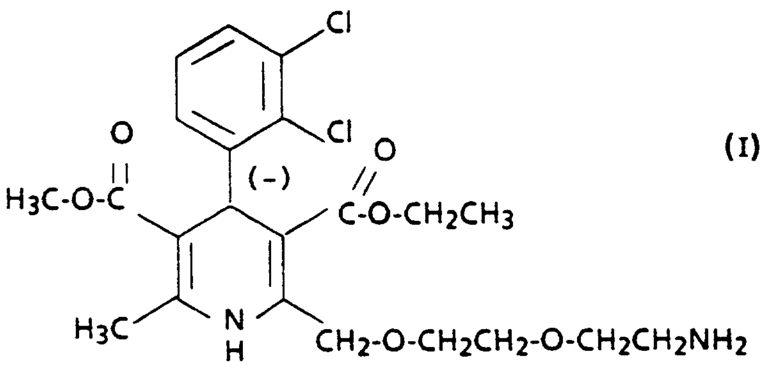

(I)

caractérisé en ce que l'on condense le [(chloro-2 éthoxy)-2 éthoxy]-2 éthanol avec la phtalimide potassique, dans la diméthylformamide à chaud, pour former le [(phtalimido-2 éthoxy)-2 éthoxy]-2 éthanol, composé de formule II,

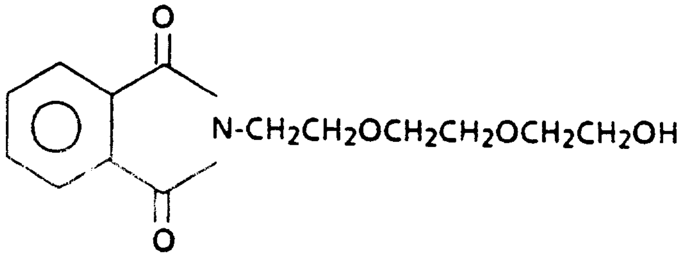

(II)

lequel ensuite est transformé à l'aide du réactif de Jones en acide [(phtalimldo-2 éthoxy)-2 éthoxy]-2 acétique, composé de formule III:

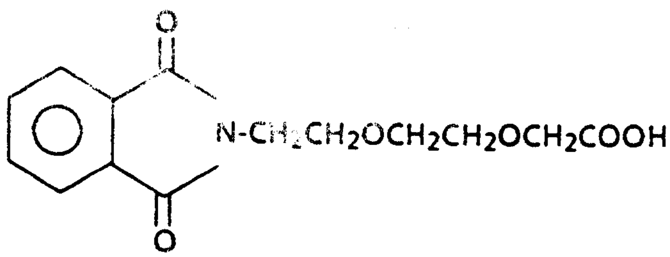

(III)

que l'on traite avec le carbonyldiimidazole et l'acide de Meldrum en présence de pyridine dans le chlorure de méthylène, pour obtenir le composé de formule IV:

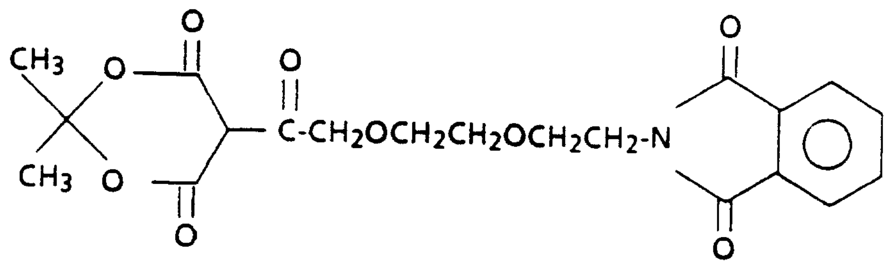

(IV)

lequel ensuite est mis à réagir avec le (R) phényl-2 méthoxy-2 éthanol, composé de formule V :

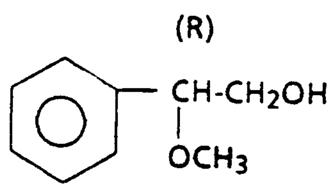

(V)

pour obtenir le $\beta$-cétoester de formule VI:

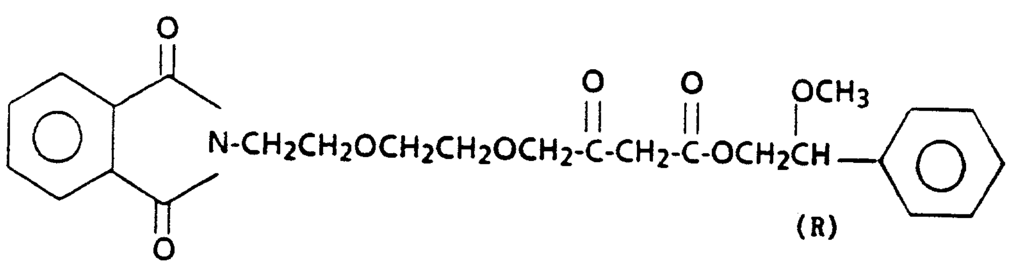

(VI)

qu'on condense en présence de formiate d'ammonium dans l'éthanol avec un benzylidène de formule VII:

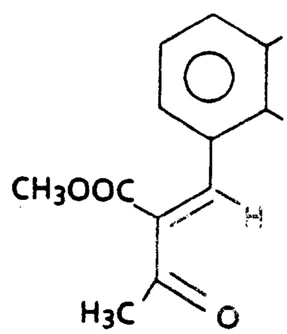

(VII)

pour obtenir la (4R4'R/4S4'R) (dichloro-2,3 phényl)-4 méthoxycarbonyl-5 (méthoxy-2 phényl-2 éthoxy-carbonyl)-3 méthyl-6 {[(phtalimido-2 éthoxy)-2 éthoxy] méthyl}-2 dihydro-1,4 pyridine, composé de formule VIII:

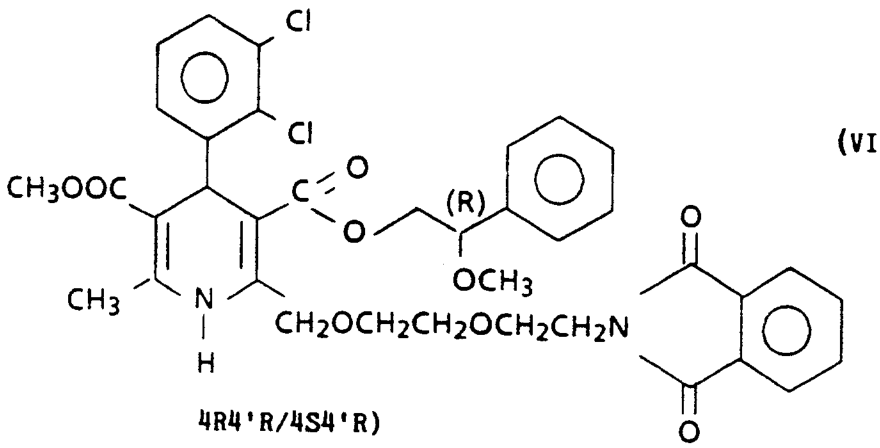

(VIII)

4R4'R/4S4'R)

laquelle ensuite est

soit:

soumise à l'action d'une solution aqueuse de bicarbonate de sodium, en solution dans l'acétonitrile, pour obtenir la (4R4'R/4S4'R) {[[(carboxy-2 phénylcarboxamido)-2 éthoxy)]-2 éthoxy] méthyl}-2 (dichloro-2,3 phényl )-4 méthoxycarbonyl-5 (méthoxy-2 phényl-2 éthoxycarbonyl)-3 méthyl-6 dihydro-1,4 pyridine, composé de formule IX:

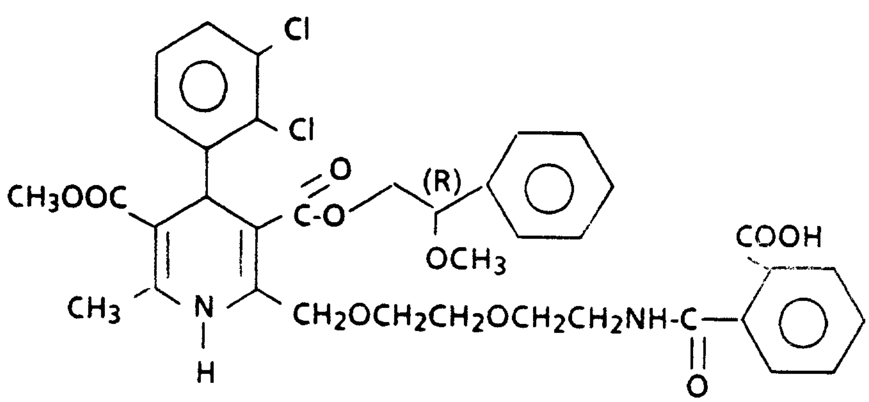

(IX)

(4R4'R/4S4'R)

que l'on traite, après séparation par HPLC, par un mélange de glyme et d'éthylate de sodium, à chaud pour obtenir un mélange contenant la (-) {[[(carboxy-2 phényl carboxamido)-2 éthoxy]-2 éthoxy] méthyl}-2 (dichloro-2,3 phényl)-4 éthoxy-carbonyl-3 méthoxycarbonyl-5 méthyl-6 dihydro-1,4 pyridine,

composé de formule X,

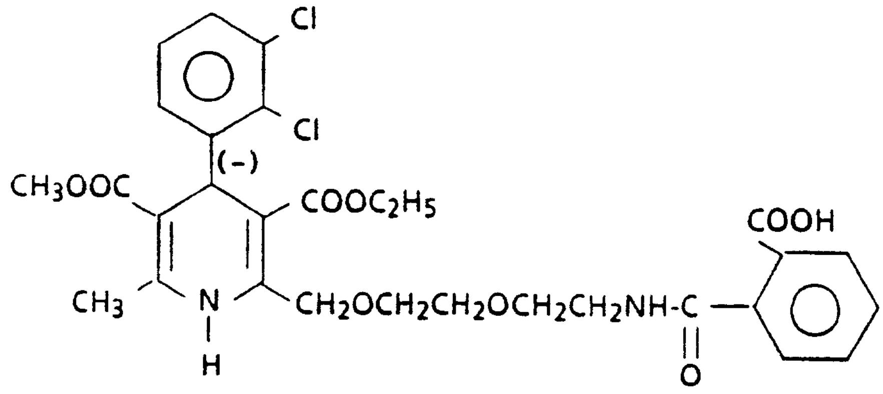

(X)

et son homologue, substitué en position 5 par un radical éthoxycarbonyle,
soit:

séparée par chromatographie sur colonne de silice en utilisant comme éluant un mélange de chlorure de méthylène et d'acétate d'éthyle (95:5 V/V) pour obtenir l'isomère le moins polaire de la (dichloro-2,3 phényl)-4 méthoxycarbonyl-5 (méthoxy-2 phényl-2 éthoxycarbonyl)-3 méthyl-6 {[-(phtalimido-2 éthoxy)-2 éthoxy] méthyl}-2 dihydro-1,4 pyridine, lequel ensuite est soumis à l'action d'éthanolate de sodium en présence de glyme en solution dans l'éthanol, pour obtenir un mélange contenant le composé de formule X et son homologue, substitué en position 5 par un radical éthoxycarbonyle, lequel est ensuite soumis à l'action du carbonyldiimidazole en solution dans un alcane halogéné à température ambiante pour obtenir un mélange contenant de la (-) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine composé de formule XI:

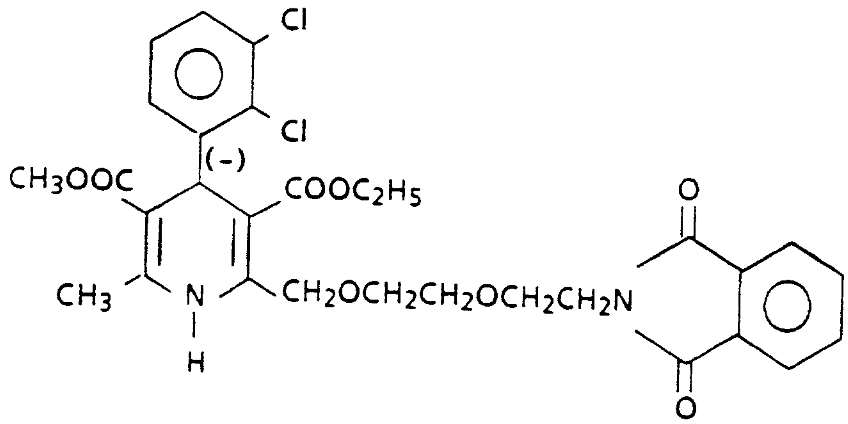

(XI)

et son homologue substitué en 5 par un radical éthoxycarbonyle lequel mélange est ensuite purifié par HPLC, (phase inverse), pour obtenir la (-) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine, laquelle est ensuite portée au reflux dans l'éthanol en présence d'hydrate d'hydrazine pour donner le composé de la formule I qui peut-être ensuite salifié avec un acide organique ou minéral pharmaceutiquement compatible.

**2.** La (4R4'R/4S4'R) (dichloro-2,3 phényl)-4 méthoxycarbonyl-5 (méthoxy-2 phényl-2 éthoxycarbonyl)-3 méthyl-6 {[(phtalimido-2 éthoxy)-2 éthoxy] méthyl}-2 dihydro-1,4 pyridine, composé de formule VIII:

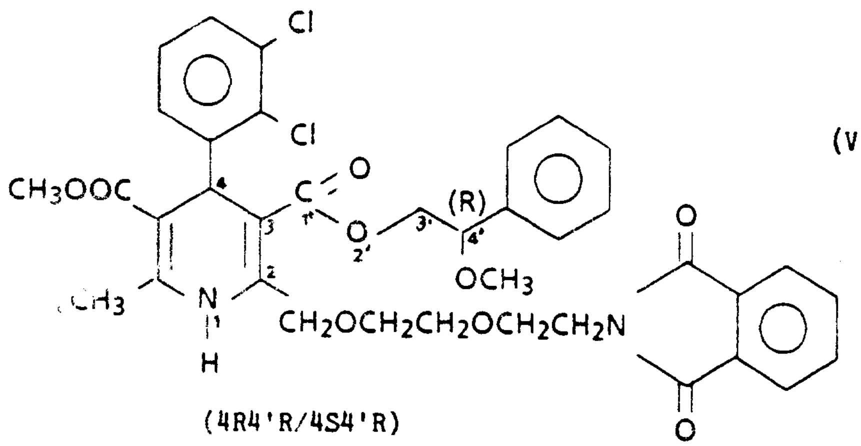

(4R4'R/4S4'R)

(VIII)

intermédiaire utile pour la préparation du composé de formule I selon la revendication 1.

**Revendication pour les Etats contractants suivants : ES, GR**

**1.** Procédé de préparation du composé de formule I :

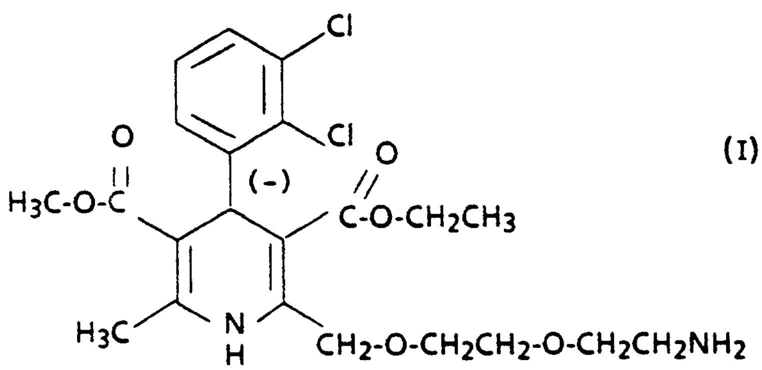

(I)

caractérisé en ce que l'on condense le [(chloro-2 éthoxy)-2 éthoxy]-2 éthanol avec la phtalimide potassique, dans la diméthylformamide à chaud, pour former le [(phtalimido-2 éthoxy)-2 éthoxy]-2 éthanol, composé de formule II,

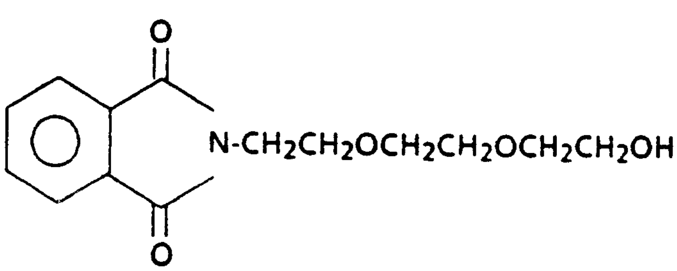

(II)

lequel ensuite est transformé à l'aide du réactif de Jones en acide [(phtalimido-2 éthoxy)-2 éthoxy]-2 acétique, composé de formule III:

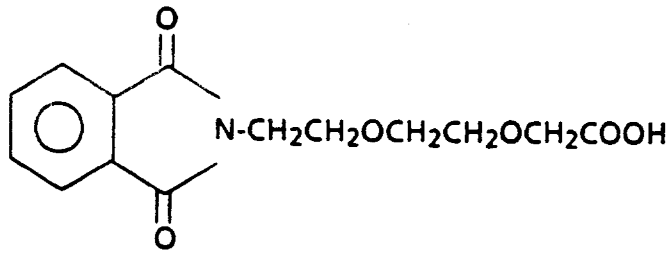

(III)

que l'on traite avec le carbonyldiimidazole et l'acide de Meldrum en présence de pyridine dans le chlorure de méthylène, pour obtenir le composé de formule IV:

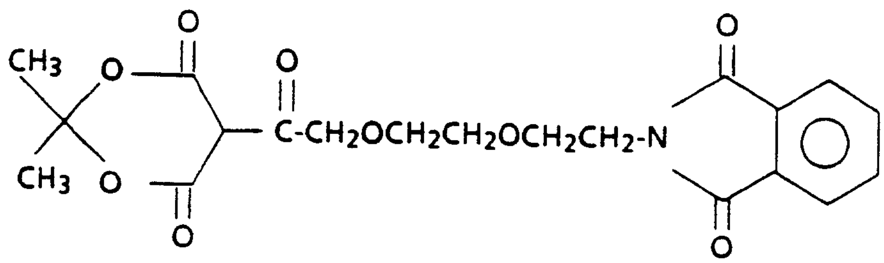

(IV)

lequel ensuite est mis à réagir avec le (R) phényl-2 méthoxy-2 éthanol, composé de formule V :

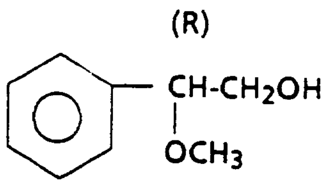

(V)

pour obtenir le $\beta$-cétoester de formule VI:

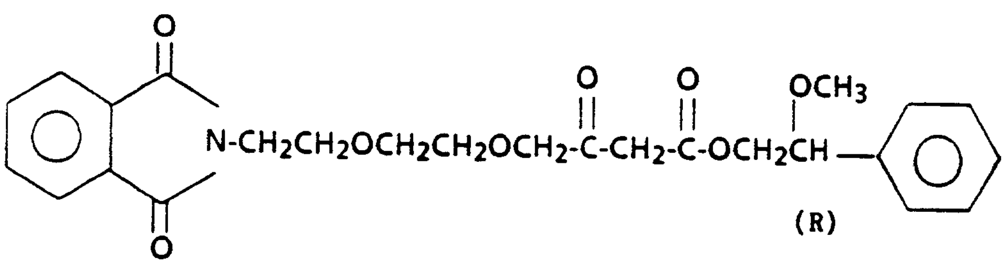

(VI)

qu'on condense en présence de formiate d'ammonium dans l'éthanol avec un benzylidène de formule VII:

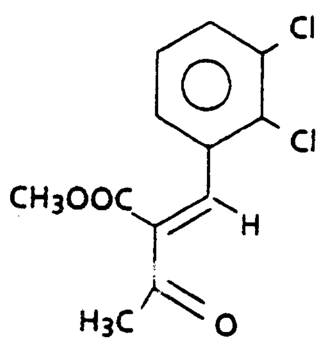

(VII)

pour obtenir la (4R4'R/4S4'R) (dichloro-2,3 phényl)-4 méthoxycarbonyl-5 (méthoxy-2 phényl-2 éthoxycarbonyl)-3 méthyl-6 {[(phtalimido-2 éthoxy)-2 éthoxy] méthyl}-2 dihydro-1,4 pyridine, composé de formule VIII:

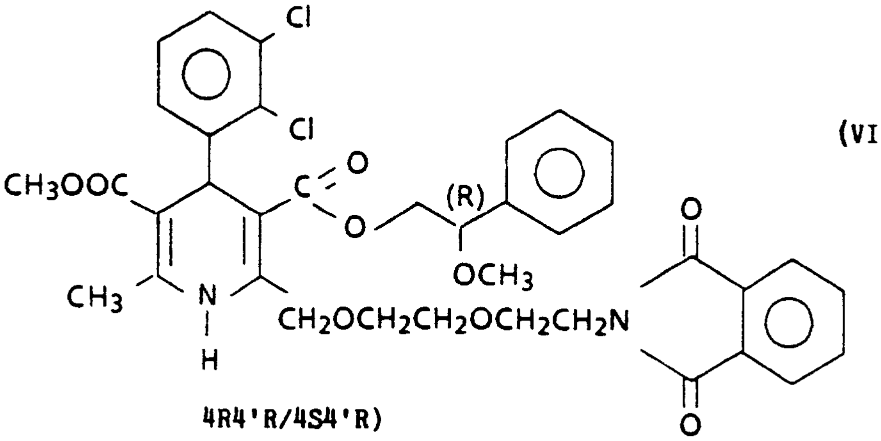

(VIII)

4R4'R/4S4'R)

laquelle ensuite est

<u>soit</u>:

soumise à l'action d'une solution aqueuse de bicarbonate de sodium, en solution dans l'acétonitrile, pour obtenir la (4R4'R/4S4'R) {[[(carboxy-2 phénylcarboxamido)-2 éthoxy)]-2 éthoxy] méthyl}-2 (dichloro-2,3 phényl)-4 méthoxycarbonyl-5 (méthoxy-2 phényl-2 éthoxycarbonyl)-3 méthyl-6 dihydro-1,4 pyridine, composé de formule IX:

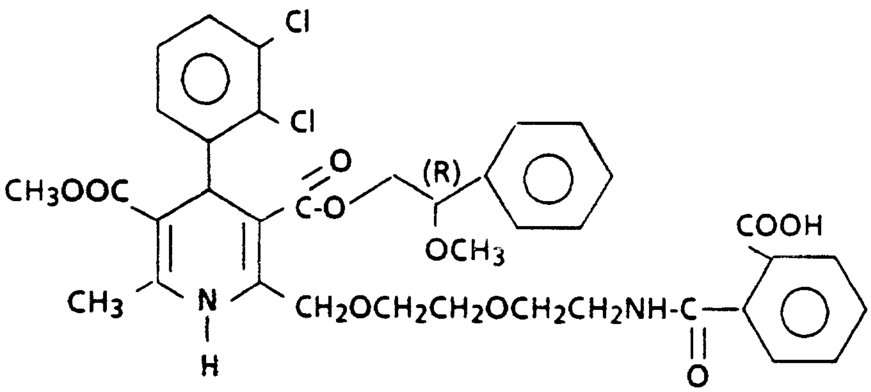

(IX)

(4R4'R/4S4'R)

que l'on traite, après séparation par HPLC, par un mélange de glyme et d'éthylate de sodium, à chaud pour obtenir un mélange contenant la (-) {[[(carboxy-2 phényl carboxamido)-2 éthoxy]-2 éthoxy] méthyl}-2 (dichloro-2,3 phényl)-4 éthoxy-carbonyl-3 méthoxycarbonyl-5 méthyl-6 dihydro-1,4 pyridine, composé de formule X,

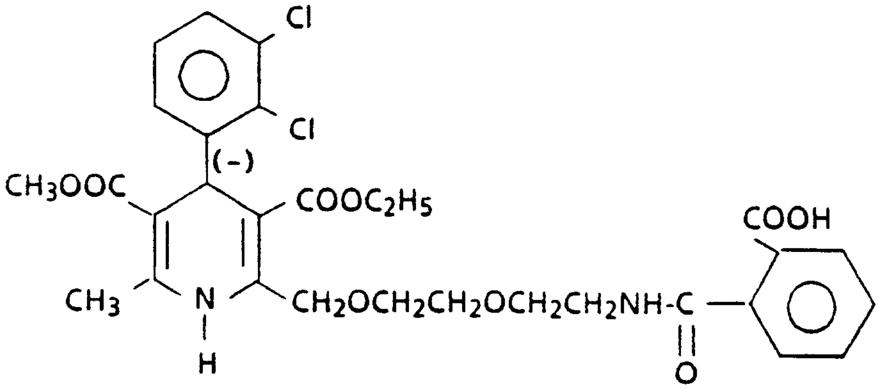

(X)

et son homologue, substitué en position 5 par un radical éthoxycarbonyle,
soit:

séparée par chromatographie sur colonne de silice en utilisant comme éluant un mélange de chlorure de méthylène et d'acétate d'éthyle (95:5 V/V) pour obtenir l'isomère le moins polaire de la (dichloro-2,3 phényl)-4 méthoxycarbonyl-5 (méthoxy-2 phényl-2 éthoxycarbonyl)-3 méthyl-6 {[-(phtalimido-2 éthoxy)-2 éthoxy] méthyl}-2 dihydro-1,4 pyridine, lequel ensuite est soumis à l'action d'éthanolate de sodium en présence de glyme en solution dans l'éthanol, pour obtenir un mélange contenant le composé de formule X et son homologue, substitué en position 5 par un radical éthoxycarbonyle, lequel est ensuite soumis à l'action du carbonyldiimidazole en solution dans un alcane halogéné à température ambiante pour obtenir un mélange contenant de la (-) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine composé de formule XI:

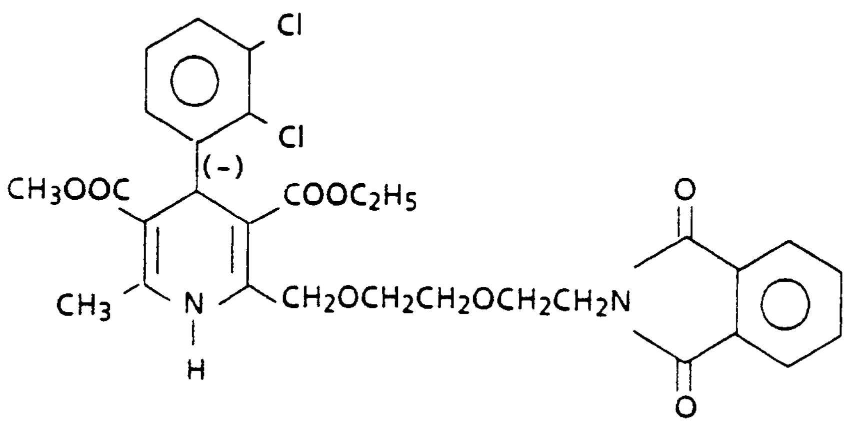

(XI)

et son homologue substitué en 5 par un radical éthoxycarbonyle lequel mélange est ensuite purifié par HPLC, (phase inverse), pour obtenir la (-) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine, laquelle est ensuite portée au reflux dans l'éthanol en présence d'hydrate d'hydrazine pour donner le composé de la formule I qui peut-être ensuite salifié avec un acide organique ou minéral pharmaceutiquement compatible.

## Claims

**Claims for the following Contracting States : AT, BE, CH, DE, FR, GB, IT, LI, LU, NL, SE**

**1.** Process for the preparation of the compound of formula I:

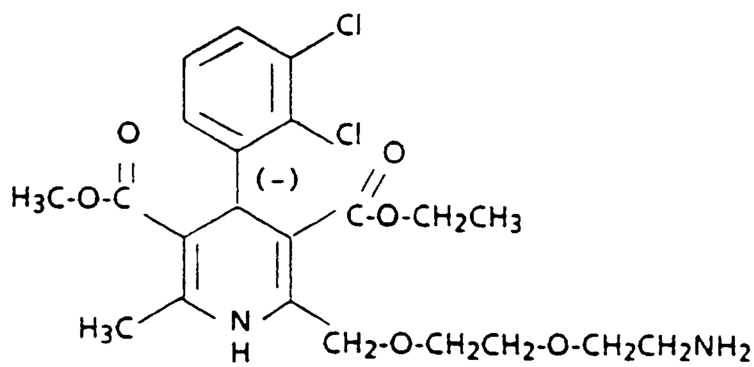

(I),

characterised in that 2-[2-(2-chloroethoxy)ethoxy]ethanol is condensed with potassium phthalimide, in dimethylformamide, with heating, to form 2-[2-(2-phthalimidoethoxy)ethoxy]ethanol, the compound of formula II:

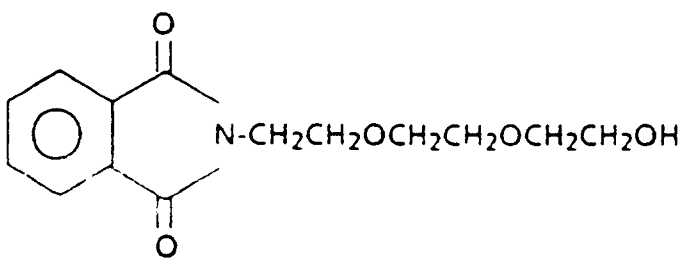

(II),

which is then converted with the aid of the Jones' reagent into 2-[2-(2-phthalimidoethoxy)ethoxy]acetic acid, the compound of formula III:

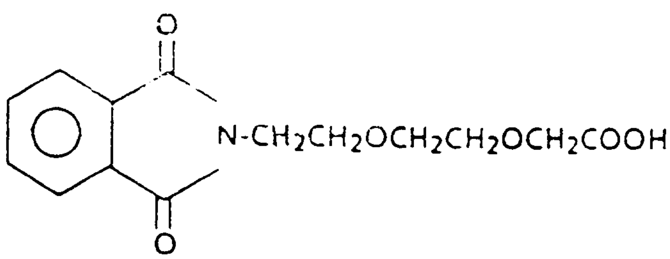

(III),

which is treated with carbonyldiimidazole and Meldrum acid in the presence of pyridine in methylene chloride, to obtain the compound of formula IV:

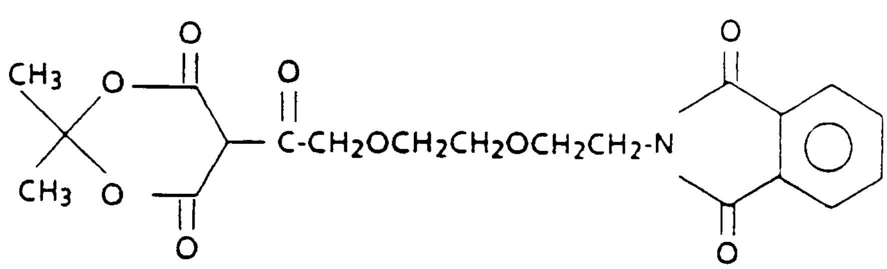

(IV),

which is then reacted with (R)-2-phenyl-2-methoxyethanol, the compound of formula V:

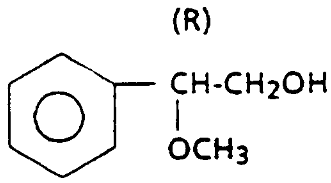

(V),

to obtain the β-keto ester of formula VI:

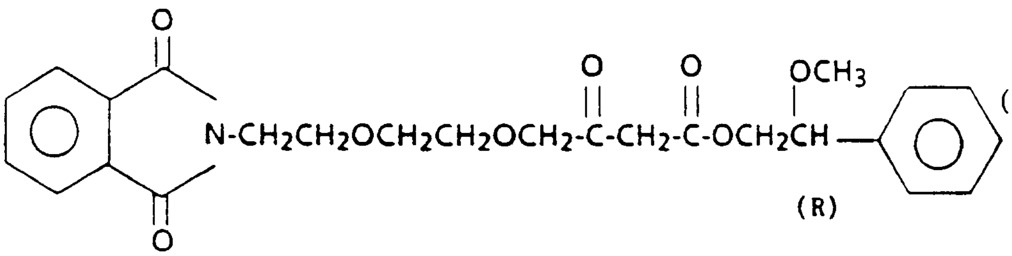

(VI),

which is condensed in the presence of ammonium formate in ethanol, with a benzylidene of formula VII:

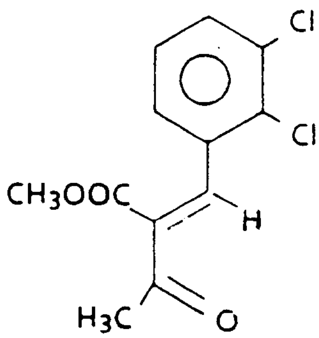

(VII)

to obtain (4R,4'R/4S,4'R)-4-(2,3-dichlorophenyl)-5-methoxycarbonyl-3-(2-methoxy-2-phenylethoxycarbonyl)-6-methyl-2-{[2-(2-phthalimidoethoxy)ethoxy]methyl}-1,4-dihydropyridine, the compound of formula VIII:

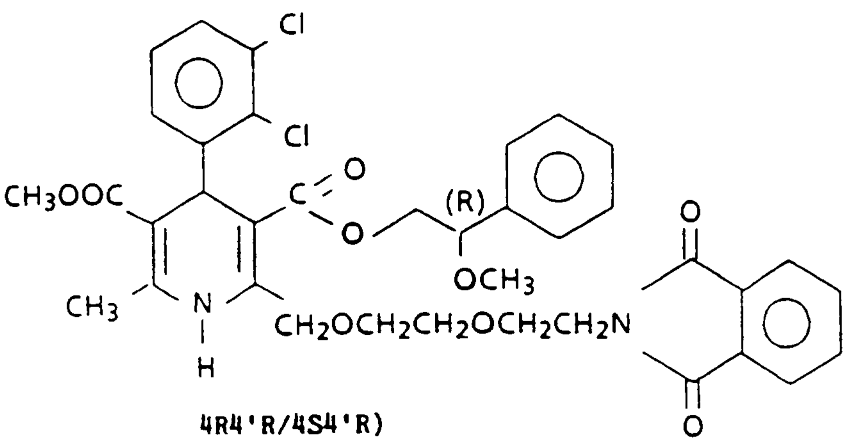

(VIII),

which is then
<u>either</u>:

subjected to the action of an aqueous solution of sodium hydrogen carbonate, in solution in acetonitrile, to obtain (4R,4'R/4S,4'R)-2-{[2-[2-(2-carboxyphenylcarboxamido)ethoxy]ethoxy]methyl}-4-(2,3-dichlorophenyl)-5-methoxycarbonyl-3-(2-methoxy-2-phenylethoxycarbonyl)-6-methyl-1,4-dihydropyridine, the compound of formula IX:

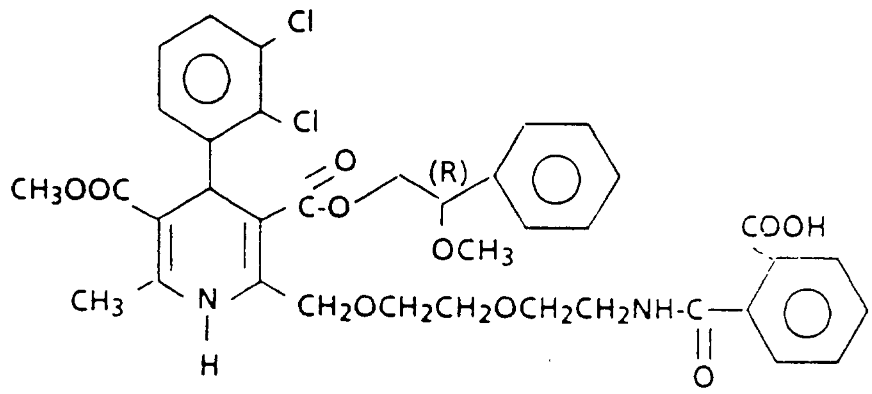

(4R4'R/4S4'R)

(IX),

which is treated, after separation by HPLC, with a mixture of glyme and sodium ethoxide, with heating, to obtain a mixture containing (-)-2-{[2-[2-(2-carboxyphenylcarboxamido)ethoxy]ethoxy]methyl}-4-(2,3-dichlorophenyl)-3-ethoxycarbonyl-5-methoxycarbonyl-6-methyl-1,4-dihydropyridine, the compound of formula X:

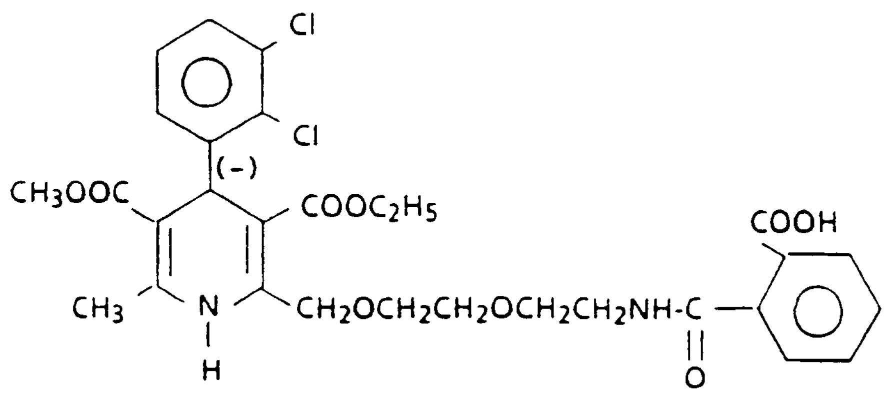

(X),

and its homologue substituted in the 5-position by an ethoxycarbonyl radical,
or:
separated by chromatography on a silica column using as eluant a mixture of methylene chloride and ethyl acetate (95:5 v/v) to obtain the least polar isomer of 4-(2,3-dichlorophenyl)-5-methoxycarbonyl-3-(2-methoxy-2-phenylethoxycarbonyl)-6-methyl-2-{[2-(2-phthalimidoethoxy)ethoxy]methyl}-1,4-dihydropyridine, which is then subjected to the action of sodium ethoxide in the presence of glyme in solution in ethanol, to obtain a mixture containing the compound of formula X and its homologue substituted in the 5-position by an ethoxycarbonyl radical, which is then subjected to the action of carbonyldiimidazole in solution in a halogenated alkane at room temperature to obtain a mixture containing (-)-4-(2,3-dichlorophenyl)-3-ethoxycarbonyl-5-methoxycarbonyl-6-methyl-2-{[2-(2-phthalimidoethoxy)ethoxy]methyl}-1,4-dihydropyridine, the compound of formula XI:

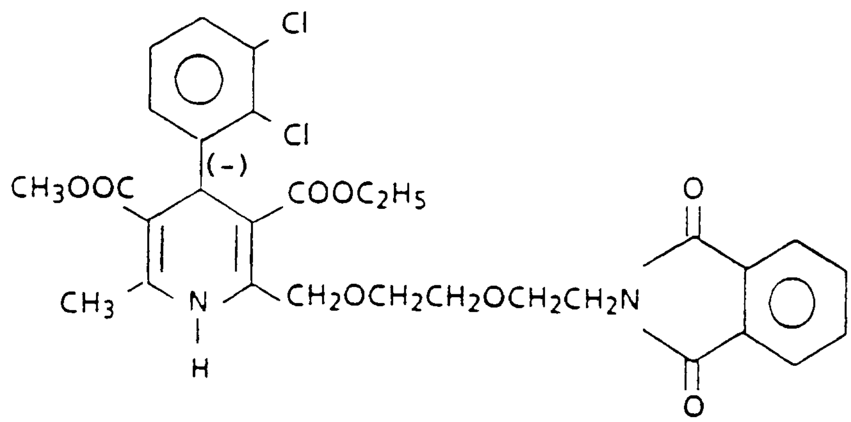

(XI),

and its homologue substituted in the 5-position by an ethoxycarbonyl radical, which mixture is then purified by HPLC (reversed phase), to obtain (-)-4-(2,3-dichlorophenyl)-3-ethoxycarbonyl-5-methoxycarbonyl-6-methyl-2-{[2-(2-phthalimidoethoxy)ethoxy]methyl}-1,4-dihydropyridine, which is then refluxed in ethanol in the presence of hydrazine hydrate to give the compound of formula I, which may then be converted into a salt with a pharmaceutically compatible organic or mineral acid.

2. (4R,4'R/4S,4'R)-4-(2,3-dichlorophenyl)-5-methoxycarbonyl-3-(2-methoxy-2-phenylethoxycarbonyl)-6-methyl-2-{[2-(2-phthalimidoethoxy)ethoxy]methyl}-1,4-dihydropyridine, the compound of formula VIII:

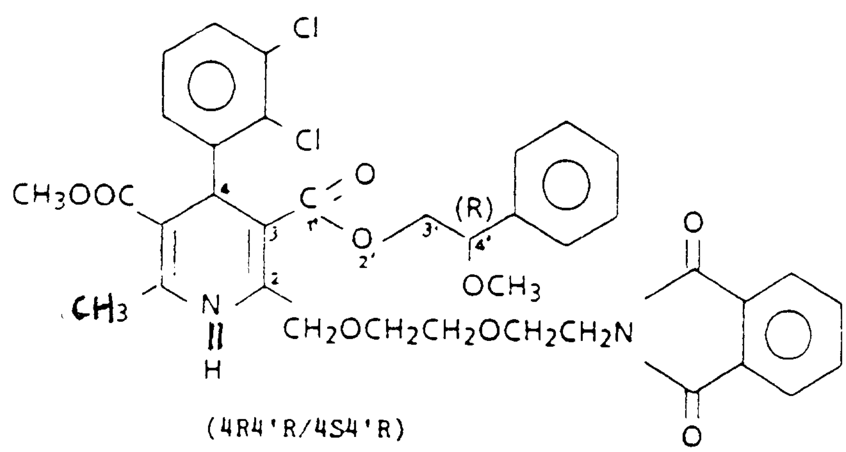

(4R4'R/4S4'R)

(VIII),

a useful intermediate for the preparation of the compound of formula I according to claim 1.

**Claim for the following Contracting States : ES, GR**

1. Process for the preparation of the compound of formula I:

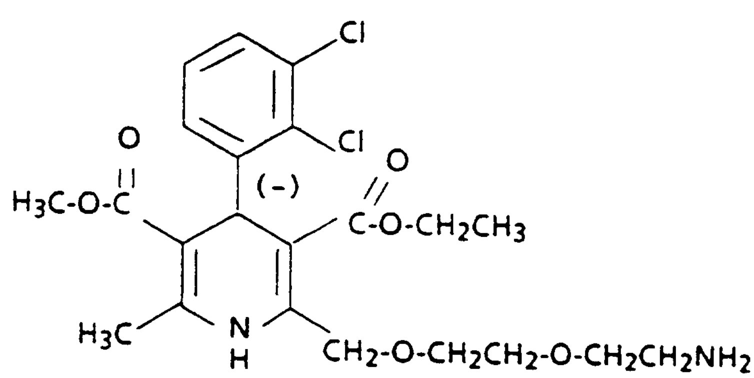

(I),

characterised in that 2-[2-(2-chloroethoxy)ethoxy]ethanol is condensed with potassium phthalimide, in dimethylformamide, with heating, to form 2-[2-(2-phthalimidoethoxy)ethoxy]ethanol, the compound of formula II:

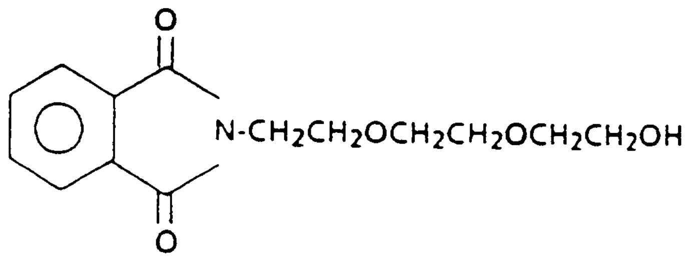

(II),

which is then converted with the aid of the Jones' reagent into 2-[2-(2-phthalimidoethoxy)ethoxy]acetic acid, the compound of formula III:

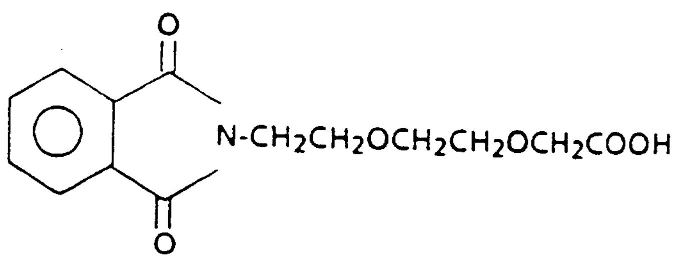

(III),

which is treated with carbonyldiimidazole and Meldrum acid in the presence of pyridine in methylene chloride, to obtain the compound of formula IV:

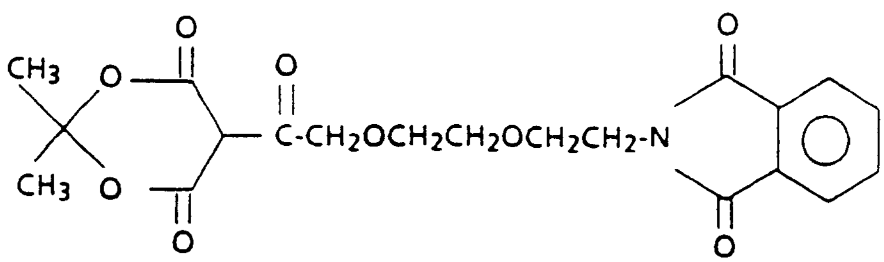

(IV),

which is then reacted with (R)-2-phenyl-2-methoxyethanol, the compound of formula V:

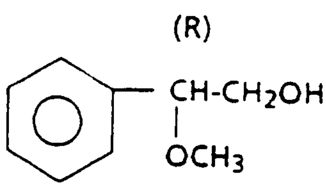

(V),

to obtain the β-keto ester of formula VI:

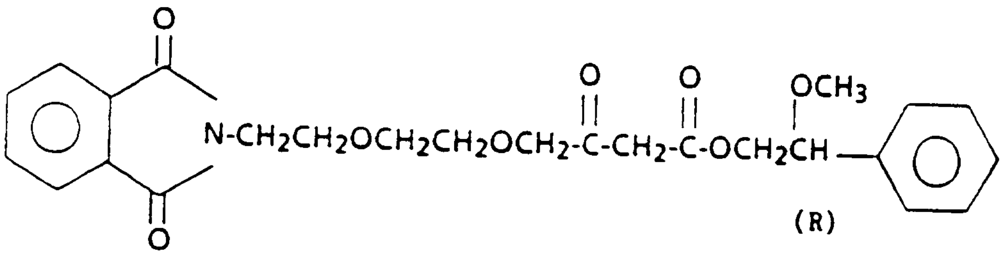

(VI),

which is condensed in the presence of ammonium formate in ethanol, with a benzylidene of formula VII:

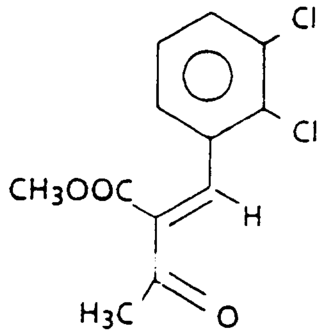

(VII)

to obtain (4R,4'R/4S,4'R)-4-(2,3-dichlorophenyl)-5-methoxycarbonyl-3-(2-methoxy-2-phenylethoxycarbonyl)-6-methyl-2-{[2-(2-phthalimidoethoxy)ethoxy]methyl}-1,4-dihydropyridine, the compound of formula VIII:

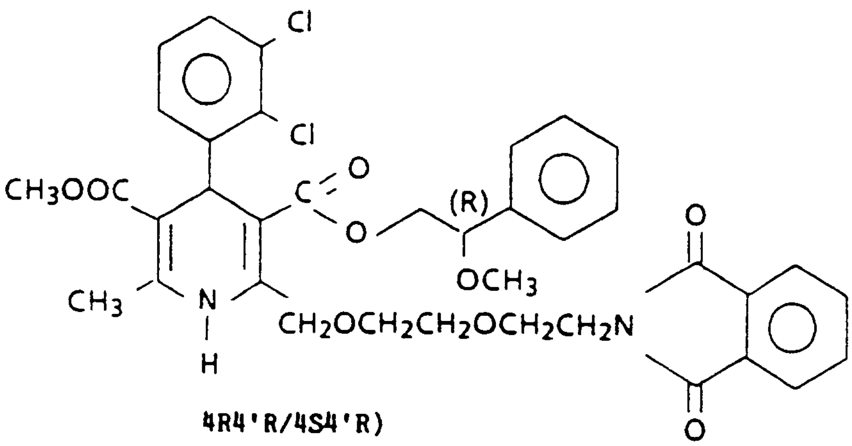

(VIII),

which is then

<u>either</u>:

subjected to the action of an aqueous solution of sodium hydrogen carbonate, in solution in acetonitrile, to obtain (4R,4'R/4S,4'R)-2-{[2-[2-(2-carboxyphenylcarboxamido)ethoxy]ethoxy]methyl}-4-(2,3-dichlorophenyl)-5-methoxycarbonyl-3-(2-methoxy-2-phenylethoxycarbonyl)-6-methyl-1,4-dihydropyridine, the compound of formula IX:

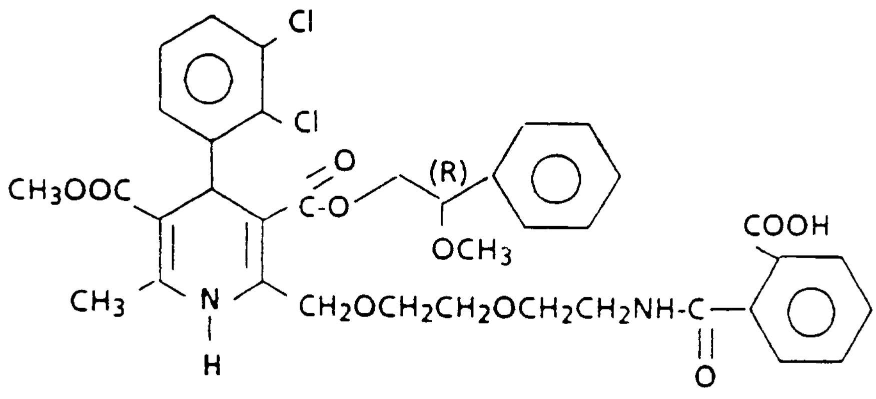

(IX),

(4R4'R/4S4'R)

which is treated, after separation by HPLC, with a mixture of glyme and sodium ethoxide, with heating, to obtain a mixture containing (-)-2-{[2-[2-(2-carboxyphenylcarboxamido)ethoxy]ethoxy]methyl}-4-(2,3-dichlorophenyl)-3-ethoxycarbonyl-5-methoxycarbonyl-6-methyl-1,4-dihydropyridine, the compound of formula X:

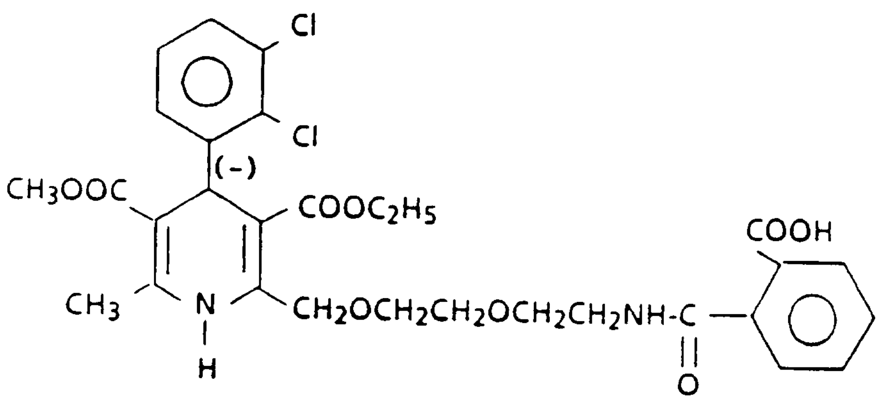

(X),

and its homologue substituted in the 5-position by an ethoxycarbonyl radical,

or:

separated by chromatography on a silica column using as eluant a mixture of methylene chloride and ethyl acetate (95:5 v/v) to obtain the least polar isomer of 4-(2,3-dichlorophenyl)-5-methoxycarbonyl-3-(2-methoxy-2-phenylethoxycarbonyl)-6-methyl-2-{[2-(2-phthalimidoethoxy)ethoxy]methyl}-1,4-dihydropyridine, which is then subjected to the action of sodium ethoxide in the presence of glyme in solution in ethanol, to obtain a mixture containing the compound of formula X and its homologue substituted in the 5-position by an ethoxycarbonyl radical, which is then subjected to the action of carbonyldiimidazole in solution in a halogenated alkane at room temperature to obtain a mixture containing (-)-4-(2,3-dichlorophenyl)-3-ethoxycarbonyl-5-methoxycarbonyl-6-methyl-2-{[2-(2-phthalimidoethoxy)ethoxy]methyl}-1,4-dihydropyridine, the compound of formula XI:

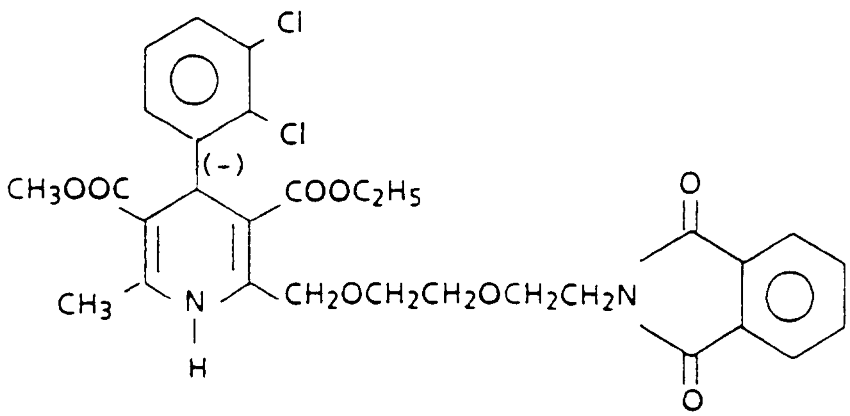

(XI),

and its homologue substituted in the 5-position by an ethoxycarbonyl radical, which mixture is then purified by HPLC (reversed phase), to obtain (-)-4-(2,3-dichlorophenyl)-3-ethoxycarbonyl-5-methoxycarbonyl-6-methyl-2-{[2-(2-phthalimidoethoxy)ethoxy]methyl}-1,4-dihydropyridine, which is then refluxed in ethanol in the presence of hydrazine hydrate to give the compound of formula I, which may then be converted into a salt with a pharmaceutically compatible organic or mineral acid.

**Patentansprüche**
**Patentansprüche für folgende Vertragsstaaten : AT, BE, CH, DE, FR, GB, IT, LI, LU, NL, SE**

**1.** Verfahren zur Herstellung der Verbindung der Formel I:

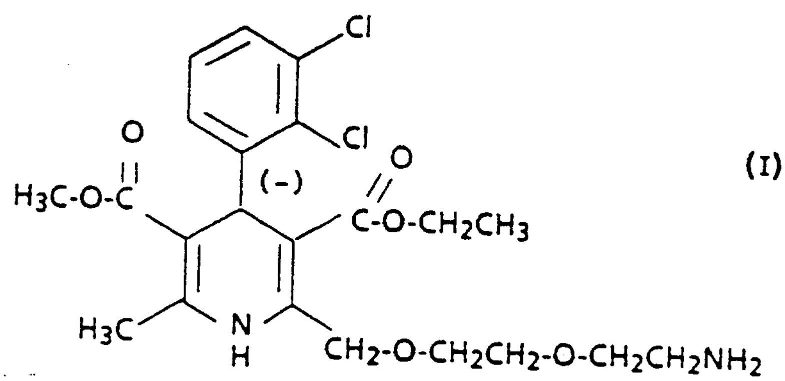

(I)

**dadurch gekennzeichnet,** daß man 2-[2-(2-Chlor-ethoxy)-ethoxy]-ethanol mit Kaliumphthalimid in warmem Dimethylformamid umsetzt zur Bildung von 2-[2-(2-Phthalimido-ethoxy)-ethoxy]-ethanol der Formel II:

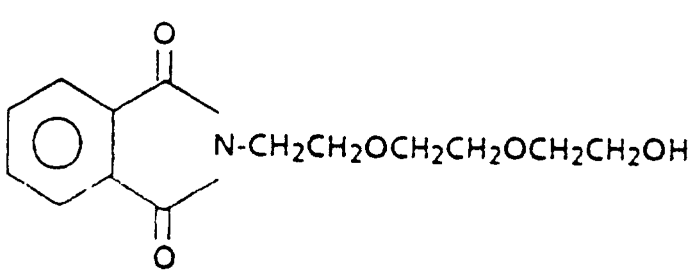

(II)

welches anschließend mit Hilfe des Jones-Reagens in die 2-[2-(2-Phthalimidoethoxy)-ethoxy]-essigsäure der Formel III umgewandelt wird:

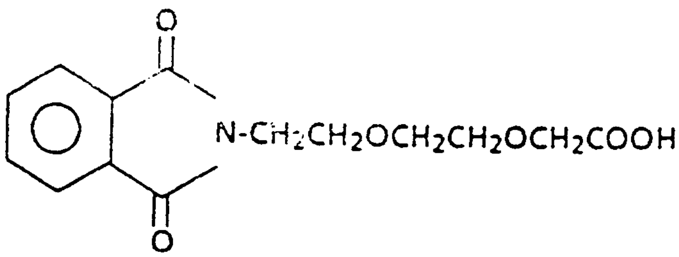

(III)

welche man Carbonyldiimidazol und Meldrum-Säure in Gegenwart von Pyridin in Methylenchlorid behandelt zur Bildung der Verbindung der Formel IV:

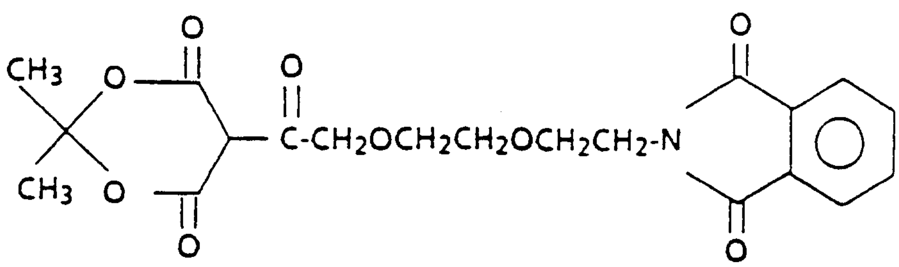

(IV)

welche anschließend mit (R)-2-Phenyl-2-methoxy-ethanol der Formel V:

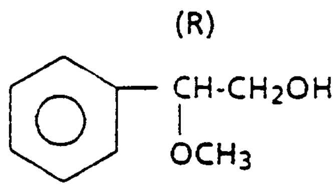

(V)

umgesetzt wird zur Bildung des $\beta$-Ketoesters der Formel VI:

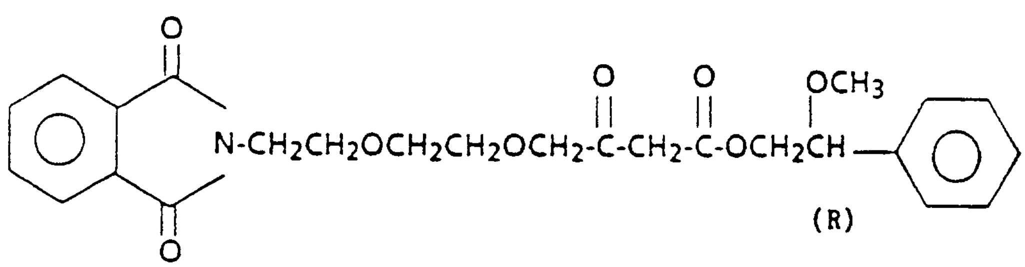

(VI)

welchen man in Gegenwart von Ammoniumformiat in Ethanol mit einem Benzyliden der Formel VII:

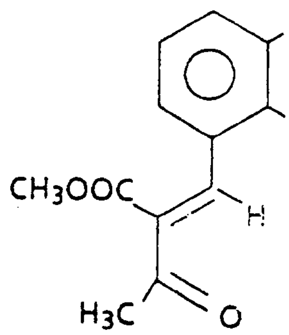

(VII)

kondensiert zur Bildung von (4R4'R/4S4'R)-4-(2,3-Dichlor-phenyl)-5-methoxycarbonyl-3-(2-methoxy-2-phenyl-ethoxycarbonyl)-6-methyl-2-{[2-(2-phthalimido-ethoxy)-ethoxy]-methyl}-1,4-dihydro-pyridin der Formel VIII:

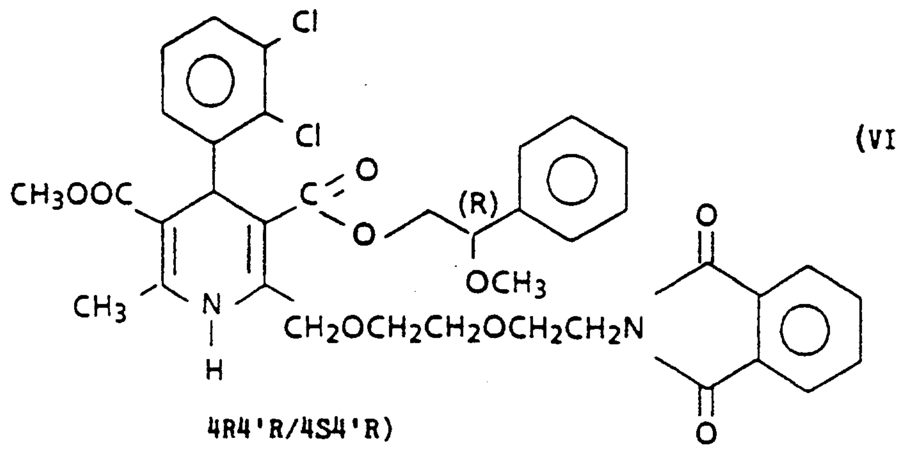

(VIII)

4R4'R/4S4'R)

welche anschließend

entweder:

in Lösung in Acetonitril der Einwirkung einer wäßrigen Natriumbicarbonatlösung unterworfen wird zur Bildung von (4R4'R/4S4'R)-2-{[2-[(2-(2-Carboxy-phenylcarboxamido)-ethoxy)] -ethoxy]-methyl}-4-(2,3-dichlor-phenyl)-5-methoxycarbonyl-3-(2-methoxy-2-phenyl-ethoxycarbonyl)-6-methyl-1,4-dihydro-pyridin der Formel IX:

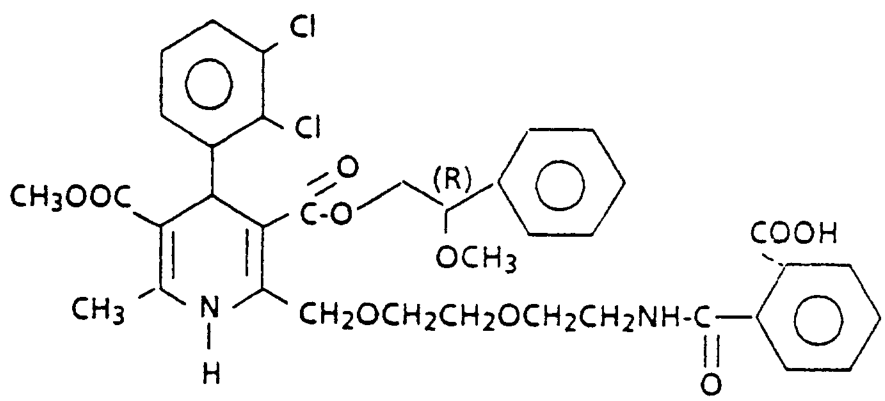

(IX)

(4R4'R/4S4'R)

welches man nach der Abtrennung durch HPLC mit einer Mischung von Glyme und Natriumethylat in der Wärme behandelt zur Bildung einer Mischung, die (-)-2-{[2-[2-(2-Carboxy-phenyl-carboxamido)-ethoxy]-ethoxy]-methyl}-4-(2,3-dichlor-phenyl)-3-ethoxycarbonyl-5-methoxycarbonyl-6-methyl-1,4-dihydro-pyridin der Formel X:

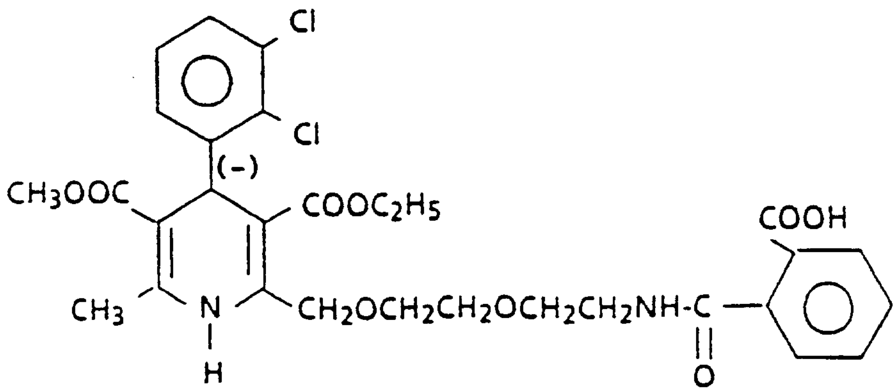

(X)

und sein in der 5-Stellung durch eine Ethoxycarbonylgruppe substituiertes Homologes enthält.
oder:
durch Säulenchromatographie über Siliciumdioxid unter Verwendung einer Mischung aus Methylenchlorid und Ethylacetat (95/5, V/V) getrennt wird zur Gewinnung des weniger polaren Isomeren von 4-(2,3-Dichlor-phenyl)-5-methoxycarbonyl-3-(2-methoxy-2-phenyl-ethoxycarbonyl)-6-methyl-2-{[2-(2-phthalimido-ethoxy)-ethoxy]-methyl}-1,4-dihydro-pyridin, welches anschließend der Einwirkung von Natriumethanolat in Gegenwart von Glyme in Lösung in Ethanol behandelt wird zur Bildung einer Mischung, welche die Verbindung der Formel X und sein in der 5-Stellung durch eine Ethoxycarbonylgruppe substituiertes Homologes enthält, welche anschließend der Einwirkung von Carbonyldiimidazol in Lösung in einem halogenierten Alkan bei Raumtemperatur unterworfen wird zur Bildung einer Mischung, die (-)-4-(2,3-Dichlor-phenyl)-3-ethoxycarbonyl-5-methoxycarbonyl-6-methyl-2-{[2-(2-phthalimido-ethoxy)-ethoxy]-methyl}-1,4-dihydro-pyridin der Formel XI:

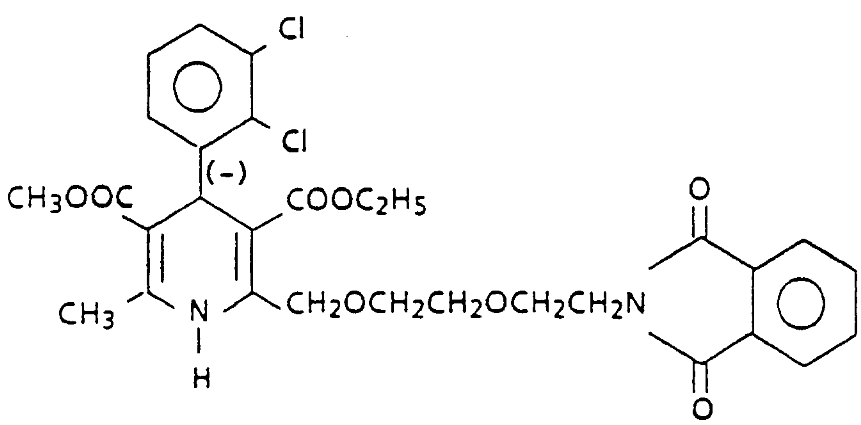

(XI)

und sein in der 5-Stellung durch eine Ethoxycarbonylgruppe substituiertes Homologes enthält, welche Mischung anschließend durch HPLC (inverse Phase) gereinigt wird unter Erhalt von (-)-4-(2,3-Dichlor-phenyl)-3-ethoxycarbonyl-5-methoxycarbonyl-6-methyl-2-{[2-(2-phthalimido-ethoxy)-ethoxy]-methyl}-1,4-di hydro-pyridin, welches anschließend in Gegenwart von Hydrazinhydrat in Ethanol zum Sieden am Rückfluß erhitzt wird zur Bildung der Verbindung der Formel I, welche anschließend mit einer organischen oder anorganischen, pharmazeutisch annehmbaren Säure in ein Salz überführt werden kann.

**2.** (4R4'R/4S4'R)-4-(2,3-Dichlor-phenyl)-5-methoxycarbonyl-3-(2-methoxy-2-phenyl-ethoxycarbonyl)-6-methyl-2-{[2-(2-phthalimido-ethoxy)-ethoxy]-methyl}-1,4-dihydro-pyridin der Formel VIII:

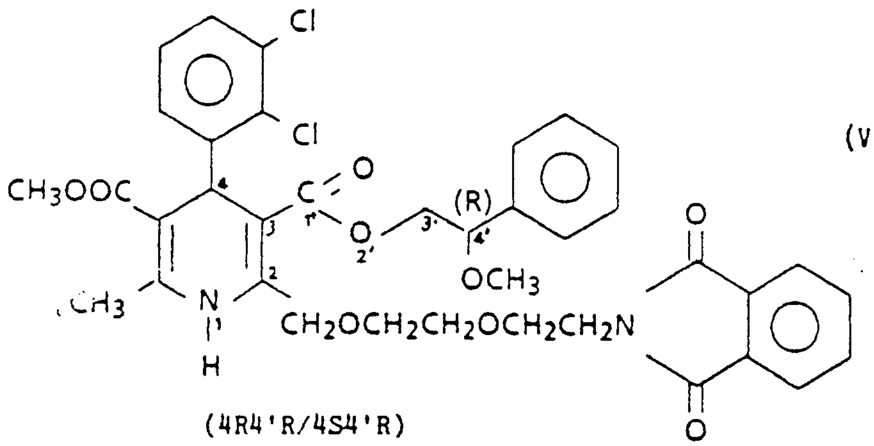

(VIII)

(4R4'R/4S4'R)

als Zwischenprodukt für die Herstellung der Verbindung der Formel I nach Anspruch 1.

**Patentanspruch für folgende Vertragsstaaten : ES, GR**

**1.** Verfahren zur Herstellung der Verbindung der Formel I:

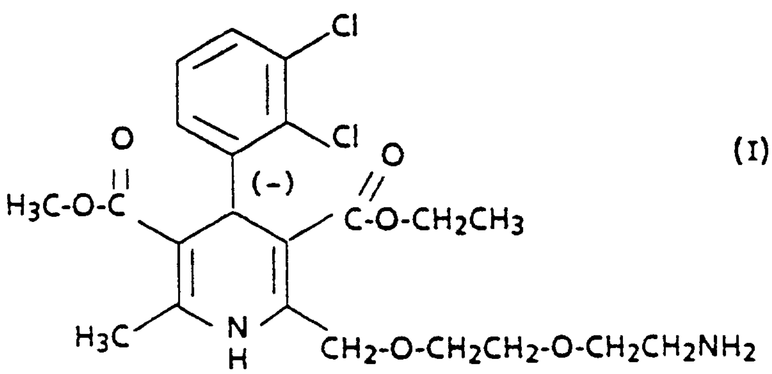

(I)

**dadurch gekennzeichnet,** daß man 2-[2-(2-Chlor-ethoxy)-ethoxyl-ethanol mit Kaliumphthalimid in warmem Dimethylformamid umsetzt zur Bildung von 2-[2-(2-Phthalimido-ethoxy)-ethoxy]-ethanol der Formel II:

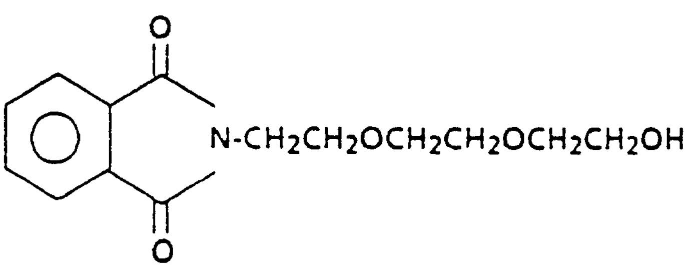

(II)

welches anschließend mit Hilfe des Jones-Reagens in die 2-[2-(2-Phthalimidoethoxy)-ethoxy]-essigsäure der Formel III umgewandelt wird:

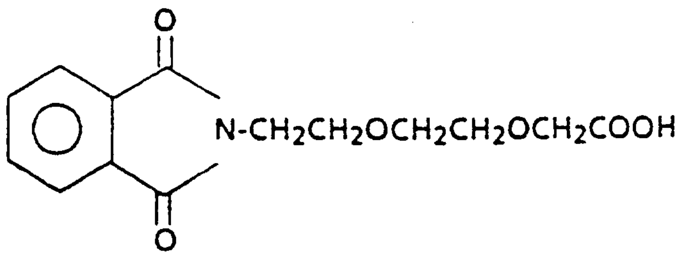

(III)

welche man Carbonyldiimidazol und Meldrum-Säure in Gegenwart von Pyridin in Methylenchlorid behandelt zur Bildung der Verbindung der Formel IV:

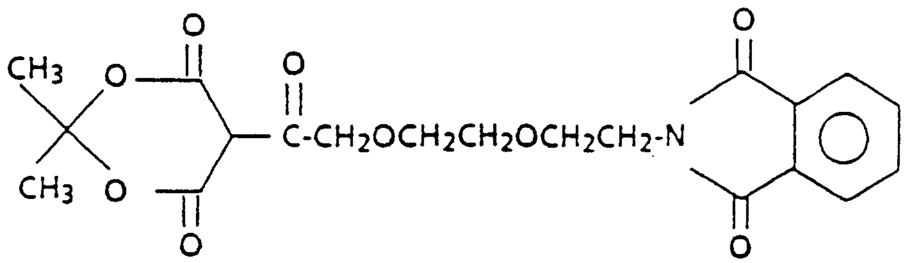

(IV)

welche anschließend mit (R)-2-Phenyl-2-methoxy-ethanol der Formel V:

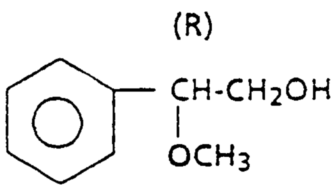

(V)

umgesetzt wird zur Bildung des $\beta$-Ketoesters der Formel VI:

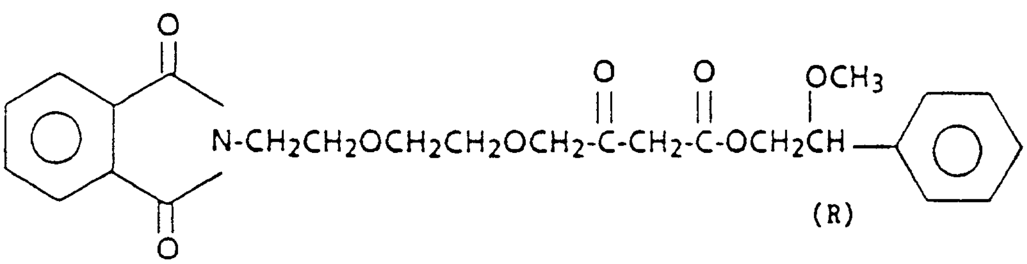

(VI)

welchen man in Gegenwart von Ammoniumformiat in Ethanol mit einem Benzyliden der Formel VII:

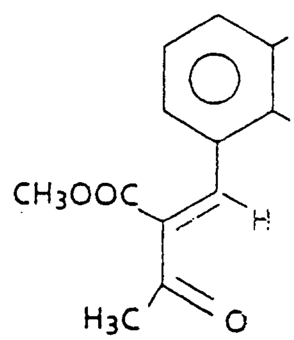

(VII)

kondensiert zur Bildung von (4R4'R/4S4'R)-4-(2,3-Dichlor-phenyl)-5-methoxycarbonyl-3-(2-methoxy-2-phenyl-ethoxycarbonyl)-6-methyl-2-{[2-(2-phthalimido-ethoxy)-ethoxy]-methyl}-1,4-dihydro-pyridin der Formel VIII:

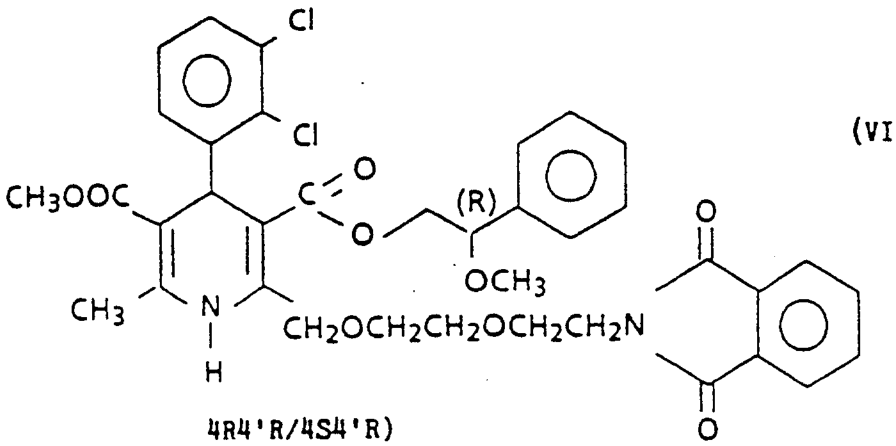

(VIII)

4R4'R/4S4'R)

welche anschließend

entweder:

in Lösung in Acetonitril der Einwirkung einer wäßrigen Natriumbicarbonatlösung unterworfen wird zur Bildung von (4R4'R/4S4'R)-2-{[2-[(2-(2-Carboxy-phenylcarboxamido)-ethoxy)]-ethoxy]-methyl}-4-(2,3-dichlor-phenyl)-5-methoxycarbonyl-3-(2-methoxy-2-phenyl-ethoxycarbonyl)-6-methyl-1,4-dihydro-pyridin der Formel IX:

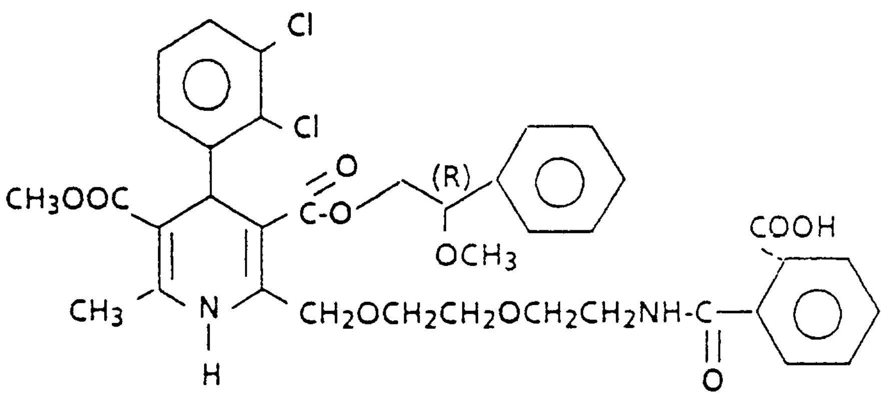

(IX)

(4R4'R/4S4'R)

welches man nach der Abtrennung durch HPLC mit einer Mischung von Glyme und Natriumethylat in der Wärme behandelt zur Bildung einer Mischung, die (-)-2-{[2-[2-(2-Carboxy-phenyl-carboxamido)-ethoxy]-ethoxy]-methyl}-4-(2,3-dichlor-phenyl)-3-ethoxycarbonyl-5-methoxycarbonyl-6-methyl-1,4-dihydro-pyridin der Formel X:

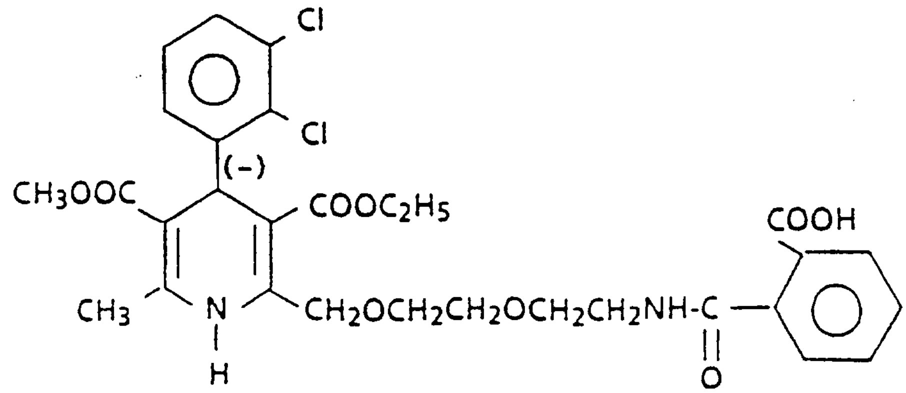

(X)

und sein in der 5-Stellung durch eine Ethoxycarbonylgruppe substituiertes Homologes enthält, oder:
durch Säulenchromatographie über Siliciumdioxid unter Verwendung einer Mischung aus Methylenchlorid und Ethylacetat (95/5, V/V) getrennt wird zur Gewinnung des weniger polaren Isomeren von 4-(2,3-Dichlor-phenyl)-5-methoxycarbonyl-3-(2-methoxy-2-phenyl-ethoxycarbonyl)-6-methyl-2-{[2-(2-phthalimido-ethoxy)-ethoxy]-methyl}-1,4-dihydro-pyridin, welches anschließend der Einwirkung von Natriumethanolat in Gegenwart von Glyme in Lösung in Ethanol behandelt wird zur Bildung einer Mischung, welche die Verbindung der Formel X und sein in der 5-Stellung durch eine Ethoxycarbonylgruppe substituiertes Homologes enthält, welche anschließend der Einwirkung von Carbonyldiimidazol in Lösung in einem halogenierten Alkan bei Raumtemperatur unterworfen wird zur Bildung einer Mischung, die (-)-4-(2,3-Dichlor-phenyl)-3-ethoxycarbonyl-5-methoxycarbonyl-6-methyl-2-{[2-(2-phthalimido-ethoxy)-ethoxy]-methyl}-1,4-dihydro-pyridin der Formel XI:

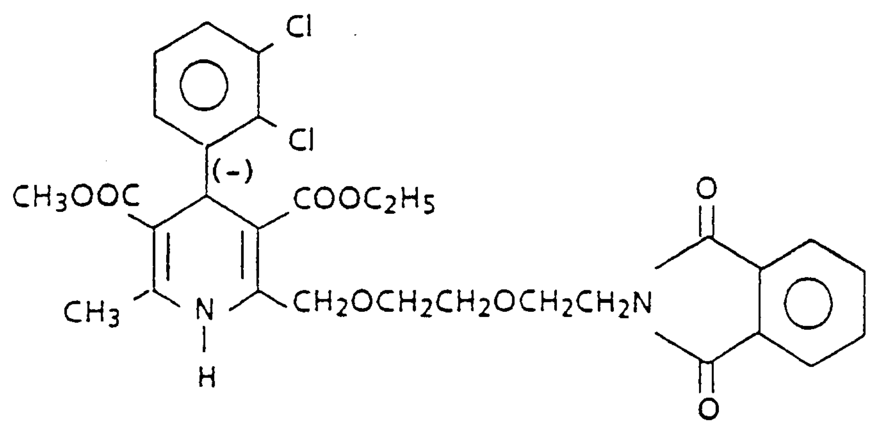

(XI)

und sein in der 5-Stellung durch eine Ethoxycarbonylgruppe substituiertes Homologes enthält, welche Mischung anschließend durch HPLC (inverse Phase) gereinigt wird unter Erhalt von (-)-4-(2,3-Dichlor-phenyl)-3-ethoxycarbonyl-5-methoxycarbonyl-6-methyl-2-{[2-(2-phthalimido-ethoxy)-ethoxy]-methyl}-1,4-di hydro-pyridin, welches anschließend in Gegenwart von Hydrazinhydrat in Ethanol zum Sieden am Rückfluß erhitzt wird zur Bildung der Verbindung der Formel I, welche anschließend mit einer organischen oder anorganischen, pharmazeutisch annehmbaren Säure in ein Salz überführt werden kann.